(19) Europäisches Patentamt
European Patent Office
Office européen des brevets

(11) EP 4 420 665 A1

(12) EUROPEAN PATENT APPLICATION
published in accordance with Art. 153(4) EPC

(43) Date of publication:
**28.08.2024 Bulletin 2024/35**

(21) Application number: **22883497.4**

(22) Date of filing: **14.10.2022**

(51) International Patent Classification (IPC):
***A61K 31/555*** *(2006.01)* ***A61K 31/409*** *(2006.01)*
***A61K 31/724*** *(2006.01)* ***A61K 47/69*** *(2017.01)*
***A61P 39/02*** *(2006.01)*

(52) Cooperative Patent Classification (CPC):
**A61K 31/409; A61K 31/555; A61K 31/724; A61K 47/69; A61P 39/02**

(86) International application number:
**PCT/JP2022/038359**

(87) International publication number:
**WO 2023/068193 (27.04.2023 Gazette 2023/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.10.2021 JP 2021170252**

(71) Applicant: **The Doshisha**
**Kyoto 602-8580 (JP)**

(72) Inventors:
- **KITAGISHI, Hiroaki**
  **Kyotanabe-shi, Kyoto 610-0394 (JP)**
- **MO, Saietsu**
  **Kyotanabe-shi, Kyoto 610-0394 (JP)**

(74) Representative: **Carstens, Dirk Wilhelm**
**Wagner & Geyer Partnerschaft mbB**
**Patent- und Rechtsanwälte**
**Gewürzmühlstraße 5**
**80538 München (DE)**

# (54) DETOXIFYING AGENT, DETOXIFYING AGENT KIT, AND NOVEL INCLUSION COMPLEX

(57) Provided are a detoxifying agent and detoxifying agent kit for simultaneous detoxification of CO and HCN in the body. The detoxifying agent for removal of carbon monoxide and hydrogen cyanide in the body includes as active ingredients a first inclusion complex wherein a first cyclodextrin dimer represented by general formula (1) below clathrates a first water-soluble metal porphyrin in which the central metal is divalent, and a second inclusion complex wherein a second cyclodextrin dimer represented by general formula (2) below clathrates the first water-soluble metal porphyrin in which the central metal is divalent, and further includes a reducing agent if necessary.

EP 4 420 665 A1

## Description

Technical Field

The present invention relates to a detoxifying agent and a detoxifying agent kit, and more specifically it relates to a detoxifying agent having a detoxifying effect for carbon monoxide and hydrogen cyanide, to a detoxifying agent kit for preparation of the detoxifying agent, and to a novel inclusion complex that can be used for the detoxifying agent.

Background Art

Death from poisoning by inhalation of toxic gas is the number one cause of death resulting from fires. The toxic components in combustion gas are primarily carbon monoxide (CO) and hydrogen cyanide (HCN). Such gas components strongly bond with heme proteins in the body such as blood hemoglobin, muscle myoglobin and cytochrome compounds in the mitochondrial intima, thereby inhibiting aerobic respiration.

The only treatment method currently available for CO poisoning is oxygen ($O_2$) ventilation. Introduction of high-purity $O_2$ gas is one method for eliminating CO from the body, but with this method it is difficult to remove CO that has infiltrated the tissues. CO poisoning also has common after effects such as cognitive impairment even after recovery.

HCN is even more toxic than CO. In Japan, amyl nitrite is used as a treatment for HCN poisoning. Amyl nitrite oxidizes blood hemoglobin from iron (II) to iron(III), and the iron(III)-converted hemoglobin strongly bonds cyanide ion, thereby alleviating toxicity. This method, however, impairs the $O_2$ transport function of hemoglobin and is therefore unsuitable for fire gas poisoning that has created a state of oxygen deficiency.

CO is generated at fire sites due to incomplete combustion of wood, while HCN gas is generated by combustion of synthetic fibers such as acrylic fibers. Both are highly toxic, but no method yet exists that can simultaneously detoxify both gases.

The present inventors have previously proposed various artificial hemoglobin model complexes using different cyclodextrin dimers.

For example, an oxygen infusion and a carbon monoxide removing agent for artificial blood have been proposed, having an inclusion complex comprising a specified cyclodextrin dimer clathrating a water-soluble metal porphyrin as the active ingredient (Patent Literatures 1 and 2).

There has also been proposed a cyanide detoxifying agent having an inclusion complex comprising a specified cyclodextrin dimer clathrated with a water-soluble metal porphyrin as the active ingredient (Patent Literature 3).

However, no practical method has yet been proposed for simultaneous detoxification of CO and HCN in the body.

Citation List

Patent Literature

Patent Literature 1: Japanese Patent Publication No. 4803631
Patent Literature 2: Japanese Published Unexamined Patent Application No. 2010-194475
Patent Literature 3: Japanese Patent Publication No. 5619500

Summary of Invention

Technical Problem

It is an object of the present invention to provide a detoxifying agent for simultaneous detoxification of CO and HCN in the body, as well as a detoxifying agent kit and novel inclusion complex.

Solution to Problem

The present inventors have conducted much ardent research with the goal of achieving the object mentioned above.

The inclusion complex described in Patent Literature 2 has high binding affinity for CO when the central metal atom is in the divalent oxidation state. In Example 1 of Patent Literature 2 as well, the central iron is reduced from the trivalent to divalent state to adjust the carbon monoxide removing agent (see paragraph [0033] of Patent Literature 2).

The inclusion complex described in Patent Literature 3 has high binding affinity for hydrogen cyanide when the

central metal atom is in the trivalent oxidation state. In Example 2 of Patent Literature 3, an inclusion complex with iron (III) is prepared as a cyanide detoxifying agent (see paragraph [0050] of Patent Literature 3). It is stated that, after adsorbing cyanide ions onto the cyanide detoxifying agent, a reducing agent was added for reduction from iron(III) to iron(II), whereby cyanide ions were rapidly dissociated (see paragraph [0054] of Patent Literature 3).

Since the inclusion complex of Patent Literature 2 and the inclusion complex of Patent Literature 3 thus have different oxidation states of the central metal atom during adsorption of toxic gas, it would seem at first that the two technologies cannot be combined.

However, the present inventors focused on the fact that the oxidation state of the inclusion complex at the time of administration and the oxidation state of the inclusion complex in the body are not necessarily the same, and set forth the novel idea of combining the respective cyclodextrin dimers of the starting materials for the inclusion complex of Patent Literature 2 and the inclusion complex of Patent Literature 3, with a water-soluble metal porphyrin and a reducing agent, whereupon after actual experimentation it was confirmed that it is thereby possible to simultaneously detoxify CO and HCN. Specifically, it was found that, when a reducing agent was administered in combination to convert each inclusion complex to the divalent state, the inclusion complex of Patent Literature 1 was not oxidized in the body, the carbon monoxide being adsorbed while still in the divalent state to allow its discharge from the body, whereas the inclusion complex of Patent Literature 3 was oxidized in the body to the trivalent state, adsorbing cyanide and allowing it to be discharged out of the body.

The present invention was completed on the basis of this finding.

More specifically, the first detoxifying agent of the present invention (hereunder referred to as "detoxifying agent 1 of the present invention") is a detoxifying agent for removal of carbon monoxide and hydrogen cyanide in the body, which includes as active ingredients a first inclusion complex wherein a first cyclodextrin dimer represented by general formula (1) below clathrates a first water-soluble metal porphyrin in which the central metal is divalent (hereunder also referred to as "hemoCDP(II)"), and a second inclusion complex as a novel inclusion complex wherein a second cyclodextrin dimer represented by general formula (2) below clathrates the first water-soluble metal porphyrin in which the central metal is divalent (hereunder also referred to as "hemoCDI(II)").

[Chemical Formula 1]

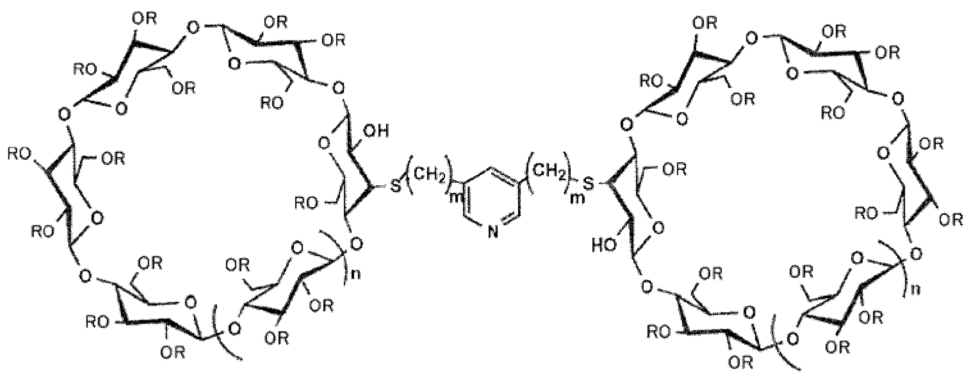

(1)

(In general formula (1) above, each R represents a protecting group protecting a hydroxyl group of cyclodextrin, m represents an integer of 1 to 2, and n represents an integer of 1 to 3.)

[Chemical Formula 2]

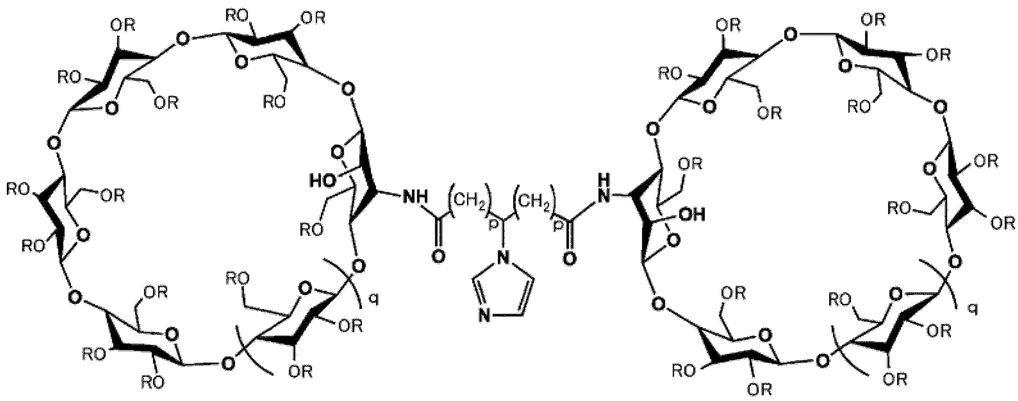

(2)

(In general formula (2) above, each R represents a protecting group protecting a hydroxyl group of cyclodextrin, p represents an integer of 1 to 2, and q represents an integer of 1 to 3.)

The detoxifying agent 1 readily undergoes oxidation of the divalent central metal, and therefore it may be mixed with a reducing agent to maintain it in a stable state for long periods.

The first detoxifying agent kit of the present invention (hereunder referred to as "detoxifying agent kit 1 of the present invention") is a detoxifying agent kit for preparing the detoxifying agent 1 of the present invention for use, and it includes an inclusion complex mixture aqueous solution containing a third inclusion complex wherein a first cyclodextrin dimer represented by general formula (1) above clathrates a water-soluble metal porphyrin in which the central metal is divalent or greater, and a fourth inclusion complex wherein the second cyclodextrin dimer represented by general formula (2) above clathrates a water-soluble metal porphyrin in which the central metal is divalent or greater, or a starting material for the same, and a reducing agent.

The second detoxifying agent of the present invention (hereunder referred to as "detoxifying agent 2 of the present invention") is a detoxifying agent for removal of carbon monoxide and hydrogen cyanide in the body, which includes as active ingredients a fifth inclusion complex as a novel inclusion complex wherein a first cyclodextrin dimer represented by general formula (1) above clathrates a second water-soluble metal porphyrin in which the central metal is trivalent (hereunder also referred to as "hemoCDP(III)"), and a sixth inclusion complex wherein a second cyclodextrin dimer represented by general formula (2) above clathrates the second water-soluble metal porphyrin in which the central metal is trivalent (hereunder also referred to as "hemoCDI(III)") .

The second detoxifying agent kit of the present invention (hereunder also referred to as "detoxifying agent kit 2 of the present invention") is a detoxifying agent kit for preparation of the aforementioned detoxifying agent 2 of the present invention for use, and it includes an inclusion complex mixture aqueous solution containing the fifth inclusion complex (hemoCDP(III)) and the sixth inclusion complex (hemoCDI(III)), or a starting material for the same.

## Advantageous Effects of Invention

With the detoxifying agent 1 or 2 of the present invention, it is possible to simultaneously detoxify CO and HCN in the body by a single administration.

The detoxifying agent kits 1 and 2 of the present invention have excellent storage stability and allow a detoxifying agent to be conveniently and rapidly prepared for use, and can be utilized at locations with highly urgent need.

## Brief Description of Drawings

[FIG. 1] FIG. 1 is a graph showing measurement results for time-dependent change in the ultraviolet-visible absorption spectrum with autoxidation of the first inclusion complex (hemoCDP(II), Reference Example 1) for Test Example 1.

[FIG. 2] FIG. 2 is a graph showing measurement results for time-dependent change in the ultraviolet-visible absorption spectrum with autoxidation of the second inclusion complex (hemoCDI(II), Reference Example 2) for Test Example 1.

[FIG. 3] FIG. 3 is a graph showing measurement results for time-dependent change in the ultraviolet-visible absorption spectrum with autoxidation of hemoCD-Twins having a mixture of two inclusion complexes (Example 1), for Test Example 1.

[FIG. 4] FIG. 4 is a graph showing results of measurement of change in the ultraviolet-visible absorption spectrum with successive addition of CO gas and NaCN to hemoCD-Twins having a mixture of two inclusion complexes (Example 1), for Test Example 2.

[FIG. 5] FIG. 5 is a graph showing results of measurement of change in the ultraviolet-visible absorption spectrum with successive addition of NaCN and CO gas to hemoCD-Twins having a mixture of two inclusion complexes (Example 1), for Test Example 2.

[FIG. 6] FIG. 6 is a graph representing three ultraviolet-visible absorption spectra for Test Example 3: the ultraviolet-visible absorption spectrum for urine of mice administered the first inclusion complex hemoCDP(II) (Reference Example 1), the ultraviolet-visible absorption spectrum after introduction of CO gas into the urine, and the ultraviolet-visible absorption spectrum after further addition of a reducing agent.

[FIG. 7] FIG. 7 is a graph representing three ultraviolet-visible absorption spectra for Test Example 3: the ultraviolet-visible absorption spectrum for urine of mice administered the second inclusion complex hemoCDI(II) (Reference Example 2), the ultraviolet-visible absorption spectrum after introduction of CO gas into the urine, and the ultraviolet-visible absorption spectrum after further addition of a reducing agent.

[FIG. 8] FIG. 8 is a graph representing three ultraviolet-visible absorption spectra for Test Example 3: the ultraviolet-visible absorption spectrum for urine of mice administered hemoCD-Twins having a mixture of hemoCDP(II) and hemoCDI(II) (Example 1), the ultraviolet-visible absorption spectrum after introduction of CO gas into the urine, and the ultraviolet-visible absorption spectrum after further addition of a reducing agent.

[FIG. 9] FIG. 9 is a graph showing results of quantifying urinary excretion of hemoCD-Twins (hourly excretion), based on the ultraviolet-visible absorption spectrum for urine of rats administered the hemoCD-Twins (Example 1), for Test Example 4.

[FIG. 10] FIG. 10 is a graph showing results of quantifying urinary excretion of hemoCD-Twins (cumulative excretion), based on the ultraviolet-visible absorption spectrum for urine of rats administered the hemoCD-Twins (Example 1), for Test Example 4.

[FIG. 11] FIG. 11is a graph showing change in heartbeat of rats after administration of the hemoCD-Twins (Example 1), for Test Example 5.

[FIG. 12] FIG. 12 is a graph showing change in blood pressure of rats after administration of the hemoCD-Twins (Example 1), for Test Example 5.

[FIG. 13] FIG. 13 is a chromatogram showing change in blood concentration in rats after administration of the hemoCD-Twins (Example 1), for Test Example 6.

[FIG. 14] FIG. 14 is a graph showing time-dependent change in blood concentration in rats after administration of the hemoCD-Twins (Example 1), for Test Example 6.

[FIG. 15] FIG. 15 is a diagram illustrating the test method of Test Example 7.

[FIG. 16] FIG. 16 is a graph showing the survival curve for rats administered NaCN and/or CO, for Test Example 7.

[FIG. 17] FIG. 17 is a graph showing behavioral observation results of rats administered NaCN and/or CO, for Test Example 7.

[FIG. 18] FIG. 18 is a diagram illustrating the test method of Test Example 8.

[FIG. 19] FIG. 19 is a graph showing the survival curve for rats following administration of 0.15 mg NaCN and 5000 ppm CO, in a group administered the hemoCD-Twins (Example 1) and in a non-administered group, for Test Example 8.

[FIG. 20] FIG. 20 is a graph showing behavioral observation results for rats following administration of 0.15 mg NaCN and 5000 ppm CO, in a group administered the hemoCD-Twins (Example 1) and in a non-administered group, for Test Example 8.

[FIG. 21] FIG. 21 is a diagram illustrating the test method of Test Example 9.

[FIG. 22] FIG. 22 is a graph showing the survival curve for rats following administration of 0.10 mg NaCN and 5000 ppm CO, in a group administered the hemoCD-Twins (Example 1) and in a non-administered group, for Test Example 9.

[FIG. 23] FIG. 23 is a graph showing behavioral observation results for rats following administration of 0.10 mg NaCN and 5000 ppm CO, in a group administered the hemoCD-Twins (Example 1) and in a non-administered group, for Test Example 9.

[FIG. 24] FIG. 24 is a graph showing change in blood pressure of rats following administration of 0.15 mg NaCN and 5000 ppm CO to the rats, in a group administered the hemoCD-Twins (Example 1) and in a non-administered group, for Test Example 10.

[FIG. 25] FIG. 25 is a graph showing time-dependent change in CO-Hb of rats following administration of 2000 ppm CO to the rats, in a group administered the hemoCD-Twins (Example 1) and in a non-administered group, for Test Example 11.

[FIG. 26] FIG. 26 is a graph showing time-dependent change in blood CN- concentration of rats following administration of 5 mg NaCN to the rats per 1 kg body weight, in a group administered the hemoCD-Twins (Example 1) and in a non-administered group, for Test Example 12.

[FIG. 27] FIG. 27 is a graph showing time-dependent change in blood concentration of rats after administration of the hemoCD-Twins (Example 1), for Test Example 13.

[FIG. 28] FIG. 28 is a urine clearance profile plotting percentage of detected hemoCD-Twins with respect to administered dose, based on UV-vis quantification of the hemoCD-Twins in urine sampled every 10 minutes after administration of the hemoCD-Twins (Example 1) to healthy rats through the femoral vein, against urine collection time, for Test Example 14.

[FIG. 29] FIG. 29 is a pair of graphs showing time-dependent change in heart rate and blood pressure before and after administration of the hemoCD-Twins (Example 1) through the femoral vein over a period of 30 minutes, for Test Example 15.

[FIG. 30] FIG. 30 is a bar graph showing a comparison between the results of measuring creatinine levels in blood sampled 24 hours after administration of the hemoCD-Twins (Example 1) to healthy mice through the femoral vein over a period of 30 minutes, and the same after administration of PBS instead of the hemoCD-Twins, for Test Example 16.

[FIG. 31] FIG. 31 is a diagram illustrating the process of collecting urine 30 minutes after administration of an aqueous solution containing hemoCDP(III) as the novel inclusion complex of the present invention to mice, for Test Example 17.

[FIG. 32] FIG. 32 is a graph showing results of spectrum analysis of a CO complex, Fe(II)-oxygen complex (oxy), and oxidized Fe(III) (met) in urine collected 30 minutes after administration of an aqueous solution containing hemoCDP(III) as the novel inclusion complex of the present invention to mice, for Test Example 17.

[FIG. 33] FIG. 33 is a diagram illustrating the process of measuring CO-Hb concentration in blood every 10 minutes after administering PBS and an aqueous solution containing the novel fifth inclusion complex of the present invention, beginning 15 minutes after administration of 2000 ppm CO to mice for 15 minutes, for Test Example 18.

[FIG. 34] FIG. 34 is a graph showing a comparison between the results of measuring CO-Hb concentration in blood every 10 minutes after administering PBS and an aqueous solution containing the novel fifth inclusion complex of the present invention, beginning 15 minutes after administration of 2000 ppm CO to mice for 15 minutes, for Test Example 18.

## Description of Embodiments

Preferred embodiments of the detoxifying agent 1 and detoxifying agent kit 1 of the present invention will now be described in detail, with the understanding that the scope of the present invention is not constrained by the description, and various modifications other than those of the Examples below may be implemented as appropriate within the gist of the present invention.

[First cyclodextrin dimer]

The first cyclodextrin dimer is represented by general formula (1) below.

[Chemical Formula 3]

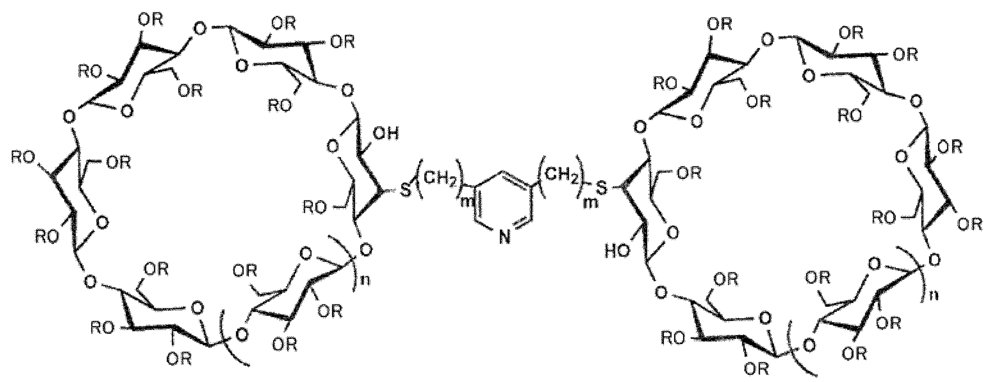

(1)

In general formula (1) above, each R represents a protecting group protecting a hydroxyl group of cyclodextrin, m represents an integer of 1 to 2, and n represents an integer of 1 to 3.

The cyclodextrin dimer represented by general formula (1) above can be produced as described in Patent Literature 1, for example, by tosylating and epoxidizing cyclodextrin, and then methylating the hydroxyl groups of cyclodextrin and bonding the methylated cyclodextrin with a linker molecule.

By protecting the hydroxyl groups of cyclodextrin beforehand with methyl groups, etc., it is possible to prevent hardening of the inner space of cyclodextrin caused by hydrogen bonds produced by the hydroxyl groups, which interferes with clathration of the water-soluble metal porphyrin in the inner space of the cyclodextrin dimer.

The protecting groups R in general formula (1) above may also be ethyl, acetyl or hydroxypropyl groups, for example, instead of methyl groups.

The cyclodextrin used as starting material for the cyclodextrin dimer represented by general formula (1) above may be α-cyclodextrin, β-cyclodextrin (n = 2) or γ-cyclodextrin, but it is preferable to use β-cyclodextrin as the starting material, with m = 1 for the linker molecule, in order to facilitate clathration of water-soluble metal porphyrin.

[Second cyclodextrin dimer]

The second cyclodextrin dimer is represented by general formula (2) below.

[Chemical Formula 4]

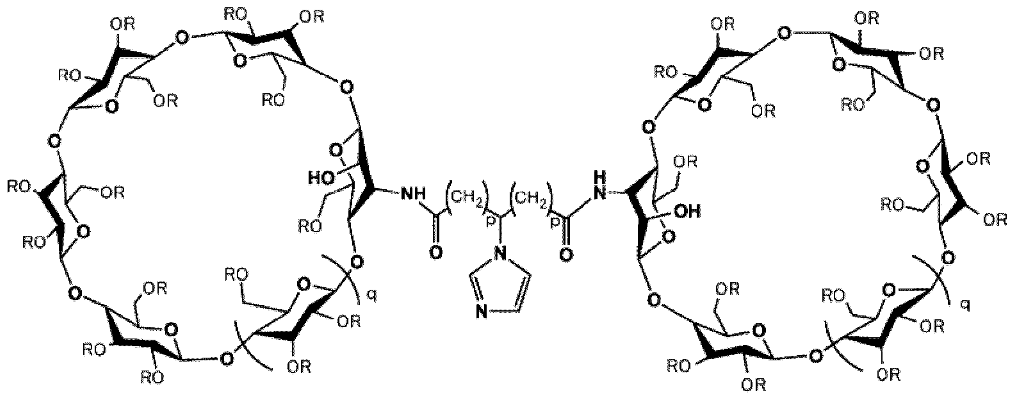

(2)

In general formula (2) above, each R represents a protecting group protecting a hydroxyl group of cyclodextrin, p represents an integer of 1 to 2, and q represents an integer of 1 to 3.

The cyclodextrin dimer represented by general formula (2) above can be produced as described in Patent Literature 3, for example, by tosylating and epoxidizing cyclodextrin, and then methylating the hydroxyl groups of cyclodextrin and bonding the methylated cyclodextrin with a linker molecule.

By protecting the hydroxyl groups of cyclodextrin beforehand with methyl groups, etc., it is possible to prevent hardening of the inner space of cyclodextrin caused by hydrogen bonds produced by the hydroxyl groups, which interferes with clathration of the water-soluble metal porphyrin in the inner space of the cyclodextrin dimer.

The cyclodextrin used as starting material for the cyclodextrin dimer represented by general formula (2) above may be α-cyclodextrin, β-cyclodextrin (q = 2) or γ-cyclodextrin, but it is preferable to use β-cyclodextrin as the starting material, with p = 1 for the linker molecule, in order to facilitate clathration of water-soluble metal porphyrin.

The protecting groups R in general formula (2) above are also not limited to methyl groups and may also be ethyl, acetyl or hydroxypropyl groups, for example.

[First water-soluble metal porphyrin]

The first water-soluble metal porphyrin is an organic compound having a cyclic structure composed of a combination of four pyrroles, with a divalent metal ion $M_1$ coordinated with the center nitrogens, and it is water-soluble.

Suitable examples include, but are not limited to, those represented by general formula (3) or (4) below.

[Chemical Formula 5]

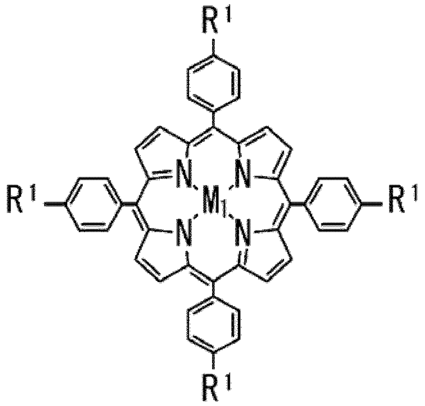

(3)

[Chemical Formula 6]

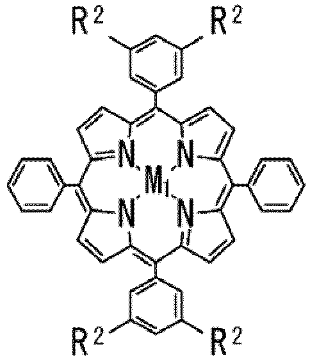

(4)

In general formulas (3) and (4) above, R1 and R2 each represent a carboxyl, sulfonyl or hydroxyl group, and $M_1$ represents $Fe^{2+}$, $Mn^{2+}$, $Co^{2+}$ or $Zn^{2+}$.

Preferable among the compounds represented by general formula (3) above is 5,10,15,20-tetrakis(4-sulfonatophenyl)porphyrin-iron complex, and preferable among the compounds represented by general formula (4) above is 5,15-bis(3,5-dicarboxylatophenyl)-10,20-diphenylporphyrin-iron complex.

These compounds may be synthesized by publicly known methods, or alternatively a commercial product (such as one by Frontier Scientific Inc., or Tokyo Kasei Kogyo Co., Ltd.) may be used directly.

In the detoxifying agent 1 of the present invention, the central metals of the first inclusion complex and second inclusion complex are divalent, but the central metal of the water-soluble metal porphyrin used to prepare the detoxifying agent may be either divalent or trivalent. When a water-soluble metal porphyrin in which the central metal is trivalent is used as the starting material for the detoxifying agent, it may be used together with a reducing agent so as to be fully reduced to the divalent state at the final stage. Since the trivalent state is generally more stable, it is more convenient to use a water-soluble metal porphyrin in which the central metal is trivalent, as the starting material for preparation of the detoxifying agent.

[Reducing agent]

Using a reducing agent can reduce the central metal of the inclusion complex to the divalent state.

Specific examples of reducing agents include sodium dithionite ($Na_2S_2O_4$), sodium ascorbate, dithiothreitol, mercaptoethanol, cysteine and glutathione.

[Detoxifying agent 1]

The detoxifying agent 1 of the present invention can be prepared in the following manner, using the first and second cyclodextrin dimers, the water-soluble metal porphyrin and the reducing agent mentioned above.

The first and second cyclodextrin dimers and the first water-soluble metal porphyrin are both water-soluble, and mixing the first and second cyclodextrin dimers and the first water-soluble metal porphyrin in an aqueous medium

can yield an inclusion complex mixture aqueous solution containing a first inclusion complex in which the central metal is divalent, wherein the first cyclodextrin dimer clathrates the first water-soluble metal porphyrin, and a second inclusion complex in which the central metal is divalent, wherein the second cyclodextrin dimer clathrates the first water-soluble metal porphyrin.

The water-soluble metal porphyrin used may also be a water-soluble metal porphyrin in which the central metal is trivalent or greater.

In such cases a reducing agent may be added and mixed in after preparation of the inclusion complex mixture aqueous solution comprising a mixture of the first and second cyclodextrin dimers and the water-soluble porphyrin, converting the central metals of the first and second inclusion complexes to their divalent states, to prepare the detoxifying agent 1 of the present invention.

The detoxifying agent 1 of the present invention may also be prepared, for example, by adding the first and second cyclodextrin dimers, the water-soluble metal porphyrin and the reducing agent to an aqueous medium, either successively or simultaneously.

A method of dissolving each of the components in an aqueous medium to prepare an aqueous solution and then combining their aqueous solutions may also be employed.

In addition, some of the starting materials may be mixed beforehand (for example, pre-mixing the powdered components before dissolution in the aqueous medium) to obtain a pre-mixture which is then mixed with the other starting materials.

The method of preparing the detoxifying agent 1 and the order of mixing the components may follow any of various patterns, with no limitation to any one.

The mixing ratio for the first and second cyclodextrin dimers and water-soluble metal porphyrin may be, stoichiometrically, water-soluble metal porphyrin:(total of first and second cyclodextrin dimers) = 1:1 (molar ratio), but this is not limitative and the total amount of the first and second cyclodextrin dimers mixed in may be in excess of the water-soluble metal porphyrin.

The mixing ratio of the first cyclodextrin dimer and second cyclodextrin dimer may be basically 1:1 (molar ratio), but the first cyclodextrin dimer content may be in slight excess if the priority is to remove carbon monoxide. Conversely, the second cyclodextrin dimer content may be in slight excess if the priority is to remove hydrogen cyanide.

The reducing agent may be added in at least the amount required to reduce the central metal of each inclusion complex to the divalent state.

An excess of the reducing agent may also be used. Even if an excess of the reducing agent remains in the detoxifying agent 1, autoxidation of the second inclusion complex proceeds once it has been diluted by administration to the body, resulting in full oxidation by the time it is excreted into the urine. This is also demonstrated by the test results of Test Example 3 below, and particularly by the test results for hemoCDI(II) (corresponding to the second inclusion complex of the detoxifying agent of the present invention) (see FIG. 7). Even when using an excess of the reducing agent, therefore, the remaining reducing agent does not need to be removed before administration of the detoxifying agent 1.

The detoxifying agent 1 of the present invention includes not only aqueous solutions with each of the components above mixed and dissolved in an aqueous medium and adjusted to suitable concentrations, but also desired dosage forms prepared with other active ingredients or additives.

The method of administration and dosage are not particularly restricted, and may be appropriately selected depending on the dosage form, and the age, body weight and degree of symptoms of the patient.

[Detoxifying agent kit 1]

The detoxifying agent kit 1 of the present invention is a kit for preparation of the detoxifying agent 1 of the present invention for use, and it includes an inclusion complex mixture aqueous solution containing the third inclusion complex and fourth inclusion complex, or starting materials for the same, and a reducing agent.

Each of these will now be explained.

<Detoxifying agent kit 1 containing inclusion complex mixture aqueous solution and reducing agent>

As mentioned above, mixing the first and second cyclodextrin dimers and the water-soluble metal porphyrin in which the central metal is divalent or greater in an aqueous medium can yield an inclusion complex mixture aqueous solution 1, containing a third inclusion complex and a fourth inclusion complex.

In the inclusion complex mixture aqueous solution, the central metal in the water-soluble metal porphyrin is divalent or greater.

The kit for the detoxifying agent of the present invention may therefore include the inclusion complex mixture aqueous solution 1 as a component of the detoxifying agent kit 1.

The inclusion complex mixture aqueous solution 1 may also be mixed with a reducing agent as another component of the detoxifying agent kit 1, thus allowing the detoxifying agent 1 to be more conveniently prepared.

<Detoxifying agent kit 1 containing starting materials for inclusion complex mixture aqueous solution, and reducing agent>

The starting materials for preparation of the inclusion complex mixture aqueous solution 1 are the components such as the first and second cyclodextrin dimers and the water-soluble metal porphyrin (optionally as an aqueous solution), or their pre-mixture (for example, a pre-mixture of the powdered components before dissolution in aqueous medium).

The first and second cyclodextrin dimers and the water-soluble metal porphyrin are all available and stable at room temperature as powders, and are thus suitable for prolonged storage when in powder form. The inclusion complex mixture aqueous solution 1 is also stable and can be stored for prolonged periods at room temperature.

From the viewpoint of long-term storage stability, therefore, the starting materials for preparation of the inclusion complex mixture aqueous solution may be used without issue in either powder form or aqueous solution form.

The starting materials for the inclusion complex mixture aqueous solution may also be mixed with a reducing agent as another component of the detoxifying agent kit 1, thus allowing the detoxifying agent 1 to be more conveniently prepared.

<Mixing ratio of components>

Adjusting the mixing ratio for each of the components as appropriate beforehand for the detoxifying agent kit 1 is preferable as this can eliminate the effort for on-site measurement and adjustment of the concentration, allowing the detoxifying agent 1 to be rapidly prepared and used. The invention is not thereby restricted, however, and the amounts of each of the components used may be adjusted as the detoxifying agent 1 is prepared, without adjustment to suitable amounts in the detoxifying agent kit 1 beforehand.

[Use of detoxifying agent 1 and detoxifying agent kit 1]

The detoxifying agent 1 of the present invention and the detoxifying agent kit 1 provide the following advantages:

- CO and HCN are simultaneously scavenged in the body and excreted into the urine;
- large-volume synthesis can be carried out at relatively low cost;
- the starting components and their solutions are highly stable and can be stockpiled at room temperature for a year or longer;
- preparation for use is fast and convenient, allowing use at locations with highly urgent need;
- an immediate effect is exhibited with a single dose; and
- they do not remain in the body and are therefore highly safe.

Given these advantages, the detoxifying agent 1 of the present invention and the detoxifying agent kit 1 offer new value in being able to provide 1) emergency treatment at fire scenes, 2) reduction in after effects of fire gas poisoning, and 3) resistance to fire gases by prior administration, as explained below, and are therefore expected to have a wide scope of potential uses.

1) Emergency treatment at fire scenes

Administration of the detoxifying agent 1 of the present invention to an animal results in rapid scavenging of CO and HCN in the body, and excretion within 1 hour. The starting material for the detoxifying agent 1 will typically be a powder reagent, which is stable at room temperature for at least one year even when dissolved in physiological saline, and can thus be stockpiled. Administration to patients that have reduced level of consciousness due to smoke inhalation at fire scenes can be expected to improve survival recovery rates from acute poisoning.

2) Reduction in after effects of fire gas poisoning

After effects induced by CO/HCN remaining in the body can be inhibited by continuous drip infusion of the detoxifying agent 1 of the present invention. Since brain dysfunction due to fire gas is inhibited by the detoxifying agent 1 of the present invention, and the level of residual CO/HCN can be measured by urinary component analysis, this can aid in judging dose adjustment and timing of dose termination.

3) Resistance to fire gases by prior administration

The detoxifying agent 1 of the present invention bonds strongly with CO/HCN by heme protein in the body, thus allowing temporary resistance to fire gases to be provided by prior administration. If it is prophylactically administered to emergency personnel beforehand, it will be possible to reduce the consequences of accidental smoke inhalation.

Preferred embodiments of the detoxifying agent 2 and the detoxifying agent kit 2 of the present invention will now be described in detail, with the understanding that the scope of the present invention is not constrained by the description, and various modifications other than those of the Examples may be implemented as appropriate within the gist of the present invention.

[Detoxifying agent 2]

Detoxifying agent 2 includes the novel inclusion complex hemoCDP(III), and hemoCDI(III).

The first cyclodextrin dimer and second cyclodextrin dimer used in the detoxifying agent 2 may be the same as for the detoxifying agent 1.

[Second water-soluble metal porphyrin]

The second water-soluble metal porphyrin is an organic compound having a cyclic structure composed of a combination of four pyrroles, with a trivalent metal ion $M_2$ coordinated with the center nitrogens, and it is water-soluble.

Suitable examples include, but are not limited to, those represented by general formula (5) or (6) below.

[Chemical Formula 7]

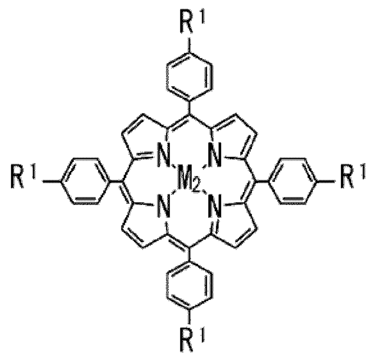

(5)

[Chemical Formula 8]

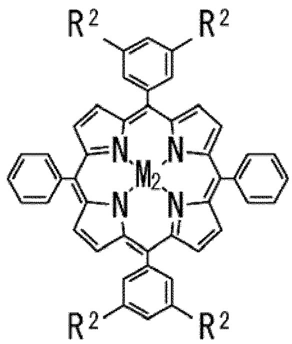

(6)

In general formulas (5) and (6) above, R1 and R2 each represent a carboxyl, sulfonyl or hydroxyl group, and $M_2$ represents $Fe^{3+}$ or $Co^{3+}$.

[Detoxifying agent kit 2]

The detoxifying agent kit 2 of the present invention is a kit for preparation of the detoxifying agent 2 of the present invention for use, and it includes the inclusion complex mixture aqueous solution 2 containing the fifth inclusion

complex and sixth inclusion complex, or starting materials for the same.

Each of these will now be explained.

<Detoxifying agent kit 2 containing inclusion complex mixture aqueous solution>

As mentioned above, mixing the first and second cyclodextrin dimers and the water-soluble metal porphyrin in which the central metal is trivalent in an aqueous medium can yield an inclusion complex mixture aqueous solution 2, containing a fifth inclusion complex and a sixth inclusion complex.

Since the central metal is trivalent in the inclusion complex mixture aqueous solution 2, it is stable and can be stored for long periods at room temperature.

The detoxifying agent kit 2 of the present invention may therefore include the inclusion complex mixture aqueous solution 2 as a component of the detoxifying agent kit 2.

<Detoxifying agent kit 2 containing inclusion complex mixture aqueous solution 2>

The starting materials for preparation of the inclusion complex mixture aqueous solution 2 are the components such as the first and second cyclodextrin dimers and the second water-soluble metal porphyrin in which the central metal is trivalent (optionally as an aqueous solution), or their pre-mixture (for example, a pre-mixture of the powdered components before dissolution in aqueous medium).

The first and second cyclodextrin dimers and the second water-soluble metal porphyrin are all available and stable at room temperature as powders, and are thus suitable for prolonged storage when in powder form. The inclusion complex aqueous solution is also stable and can be stored for prolonged periods at room temperature.

From the viewpoint of long-term storage stability, therefore, the starting materials for preparation of the inclusion complex mixture aqueous solution 2 may be used without issue in either powder form or aqueous solution form.

<Mixing ratio of components>

Adjusting the mixing ratio for each of the components as appropriate beforehand for the detoxifying agent kit 2 is preferable as this can eliminate the effort for on-site measurement and adjustment of the concentration, allowing the detoxifying agent 2 to be rapidly prepared and used. The invention is not thereby restricted, however, and the amounts of each of the components used may be adjusted as the detoxifying agent 2 is prepared, without adjustment to suitable amounts in the detoxifying agent kit 2 beforehand.

[Use of detoxifying agent 2 and detoxifying agent kit 2]

The detoxifying agent 2 of the present invention and the detoxifying agent kit 2 provide the following advantages:

- CO and HCN are simultaneously scavenged in the body and excreted into the urine;
- large-volume synthesis can be carried out at relatively low cost;
- the starting components and their solutions are highly stable and can be stockpiled at room temperature for a year or longer;
- preparation for use is fast and convenient, allowing use at locations with highly urgent need;
- an immediate effect is exhibited with a single dose; and
- they do not remain in the body and are therefore highly safe.

Given these advantages, the detoxifying agent 2 of the present invention and the detoxifying agent kit 2 offer new value in being able to provide 1) emergency treatment at fire scenes, 2) reduction in after effects of fire gas poisoning, and 3) resistance to fire gases by prior administration, as explained below, and are therefore expected to have a wide scope of potential uses.

1) Emergency treatment at fire scenes

Administration of the detoxifying agent 2 of the present invention to an animal results in rapid scavenging of CO and HCN in the body, and excretion within 1 hour. The starting material for the detoxifying agent 2 will typically be a powder reagent, which is stable at room temperature for at least one year even when dissolved in physiological saline, and can thus be stockpiled. Administration to patients that have reduced level of consciousness due to smoke inhalation at fire scenes can be expected to improve survival recovery rates from acute poisoning.

2) Reduction in after effects of fire gas poisoning

After effects induced by CO/HCN remaining in the body can be inhibited by continuous drip infusion of the detoxifying agent 2 of the present invention. Since brain dysfunction due to fire gas is inhibited by the detoxifying agent 2 of the present invention, and the level of residual CO/HCN can be measured by urinary component analysis, this can aid in judging dose adjustment and timing of dose termination.

3) Resistance to fire gases by prior administration

The detoxifying agent 2 of the present invention bonds strongly with CO/HCN by heme protein in the body, thus allowing temporary resistance to fire gases to be provided by prior administration. If it is prophylactically administered to emergency personnel beforehand, it will be possible to reduce the consequences of accidental smoke inhalation.

Preferred embodiments of the detoxifying agents 1 and 2 and the detoxifying agent kits 1 and 2 of the present invention will now be described in detail, with the understanding that the scope of the present invention is not constrained by the description, and various modifications other than those of the Examples may be implemented as appropriate within the gist of the present invention.

Examples

The detoxifying agent, detoxifying agent kit and novel inclusion complex of the present invention will now be described further using Examples, with the understanding that the present invention is not intended to be restricted by the Examples.

[Reagents]

The following reagents were used in the Examples.

The first cyclodextrin dimer used was synthesized by the cyclodextrin dimer (CD2) synthesis method described in Patent Literature 1 (see paragraphs [0016] to [0020] and FIG. 1 of Patent Literature 1). This will hereunder be referred to as "Py3CD" or "P." The specific chemical structural formula for Py3CD corresponds to general formula (1) above wherein all of the R groups are methyl groups, m is 1, and n is 2.

The second cyclodextrin dimer used was synthesized by the cyclodextrin dimer (Im3CD) synthesis method described in Patent Literature 3 (see paragraphs [0033] to [0047] and

**[0104]** FIG. 1 of Patent Literature 3). This will hereunder be referred to as "Im3CD" or "I." The specific chemical structural formula for Im3CD corresponds to general formula (2) above wherein all of the R groups are methyl groups, p is 1, and q is 2.

The water-soluble metal porphyrin used was a 5,10,15,20-tetrakis(4-sulfonatophenyl)porphyrin-iron(III) complex synthesized by a publicly known method. This compound is available as a commercial product (from Frontier Scientific Inc., for example). This will hereunder be referred to as "Fe(III)TPPS" or "F."

The reducing agent used was sodium dithionite ($Na_2S_2O_4$). This will hereunder be simply referred to as the "reducing agent."

The ultraviolet-visible absorption spectrum was measured using a spectrophotometer (UV-2600i, UV-2450, by Shimadzu Corp.).

[Example 1]

Fe(III)TPPS (F), Py3CD (P) and Im3CD (I) were mixed in a molar ratio of 2:1:1 and added to physiological saline to prepare an inclusion complex mixture aqueous solution. When 10 mL of a 3.5 mM inclusion complex mixture aqueous solution is prepared, the amounts are 40 mg of Fe(III)TPPS, 60 mg of Py3CD and 60 mg of Im3CD. When 10 mL of a 7.0 mM and 14 mM inclusion complex mixture aqueous solution is prepared, the amounts of addition are 2-fold and 4-fold, respectively.

The inclusion complex mixture aqueous solution prepared in this manner contains an inclusion complex in which Py3CD clathrates Fe(III)TPPS (corresponding to the fifth inclusion complex (hemoCDP(III)) of the present invention), and an inclusion complex in which Im3CD clathrates Fe(III)TPPS (corresponding to the sixth inclusion complex (hemoCDI(III)) of the present invention).

An excess of a reducing agent was then added to reduce the central irons of the two different inclusion complexes in the inclusion complex mixture aqueous solution from iron(III) to iron (II), to obtain the first inclusion complex (hemoCDP(II)) and second inclusion complex (hemoCDI(II)) of the present invention. The amount of reducing agent added was 5 mg/mL (about 28 mM) for each, regardless of the concentration of the inclusion complex mixture aqueous solution.

The dose of sodium dithionite used here (50 mg/kg body weight) is much lower than the value indicated for oral toxicity of sodium dithionite (2500 mg/kg body weight).

This was used as the detoxifying agent 1 for Example 1.

The detoxifying agent 1 of Example 1 may also be referred to hereunder as "hemoCD-Twins."

[Reference Example 1]

Fe(III)TPPS and Py3CD were mixed in a molar ratio of 1:1 and added to physiological saline to prepare an inclusion complex aqueous solution. When 10 mL of a 3.5 mM inclusion complex aqueous solution is prepared, the amounts are 40 mg Fe(III)TPPS and 120 mg Py3CD, and when 10 mL of 7.0 mM and 14 mM inclusion complex aqueous solutions are prepared, the amounts of addition are 2-fold and 4-fold, respectively.

An excess of a reducing agent was then added to reduce the central iron of the inclusion complex in the inclusion complex aqueous solution from iron(III) to iron(II).

This was used as the detoxifying agent for Reference Example 1.

The detoxifying agent of Reference Example 1 comprises the first inclusion complex of the present invention, and may also be referred to hereunder as "hemoCDP(II)."

[Reference Example 2]

Fe(III)TPPS and Im3CD were mixed in a molar ratio of 1:1 and added to physiological saline to prepare an inclusion complex aqueous solution. When 10 mL of a 3.5 mM inclusion complex aqueous solution is prepared, the amounts are 40 mg Fe(III)TPPS and 120 mg Im3CD, and when 10 mL of 7.0 mM and 14 mM inclusion complex aqueous solutions is prepared, the amounts of addition are 2-fold and 4-fold, respectively.

An excess of a reducing agent was then added to reduce the central iron of the inclusion complex in the inclusion complex aqueous solution from iron(III) to iron(II).

This was used as the detoxifying agent for Reference Example 2.

The detoxifying agent of Reference Example 2 comprises the sixth inclusion complex of the present invention, and may also be referred to hereunder as "hemoCDI(II)."

[Test Example 1: Measuring autoxidation reaction rate for central metal of inclusion complex]

The hemoCDP(II) synthesized in Reference Example 1 was diluted with physiological saline, and the time-dependent change in the ultraviolet-visible absorption spectrum was observed under physiological conditions (pH 7.4, 37°C) with a concentration of $4.0 \times 10^{-6}$ M in the cell. The results are shown in FIG. 1.

FIG. 1 also shows the time-dependent change in absorbance at the peak wavelength shown at top right. The change in absorbance was analyzed based on a first-order reaction rate equation.

[Formula 1]

$$A_t = (A_\infty - A_0)(1 - \exp(k_{obs} \cdot t)) + A_0 \qquad (1)$$

Here, A0 is the measuring start point, A∞ is the measuring end point, At is the absorbance at 422 nm at time t, and kobs is the pseudo-first-order rate constant for the autoxidation reaction. The obtained spectral change was analyzed by curve fitting based on formula (1), and kobs and half-life t1/2 were calculated. The values are also shown in FIG. 1.

In a similar manner for the hemoCDI(II) synthesized in Reference Example 2 as well, the time-dependent change in the ultraviolet-visible absorption spectrum was observed and the rate constant kobs and half-life t1/2 for the autoxidation reaction were calculated. The results are shown in FIG. 2.

For the hemoCD-Twins synthesized in Example 1 as well, the time-dependent change in the ultraviolet-visible absorption spectrum was observed in the same manner. The results are shown in FIG. 3.

The change in absorbance was analyzed by two-phase curve fitting.

[Formula 2]

$$A_t = (A_\infty - A_0)(1 - \exp(k_{obs} \cdot t)) + A_0 \qquad (1)$$

Here, A0 is the measuring start point, A∞ is the measuring end point, At is the absorbance at 422 nm at time t, and kfast and kslow are the respective reaction rate constants for autoxidation reaction of the two components. The

obtained spectral change was analyzed by curve fitting based on formula (2), and kfast, kslow and half-life $t_{1/2}$ were calculated.

As shown in FIG. 1, the half-life of iron (II) with hemoCDP(II) is 5.0 hours under physiological conditions (pH 7.4, 37°C), whereas with hemoCDI(II) the half-life is 36 minutes, as shown in FIG. 2. At 25°C, it is 30.1 hours for hemoCDP(II) and 3 hours for hemoCDI(II), as previously reported (Inorg. Chem. 2006; Chem. Asian J. 2006).

The autoxidation reaction with hemoCD-Twins can be analyzed with a 2nd order rate equation, and the two rate constants (kfast and kslow) respectively matched those measured for hemoCDP(II) and hemoCDI(II) alone.

Thus, it was shown that with the hemoCD-Twins synthesized in Example 1, hemoCDP(II) and hemoCDI(II) had mixed without mutual interference by their physical properties, exhibiting the same oxidation rates as each alone.

[Test Example 2: Verification of CO and CN scavenging ability of inclusion complex]

The hemoCD-Twins synthesized in Example 1 was diluted and the ultraviolet-visible absorption spectrum was measured at a concentration of $4.9 \times 10^{-6}$ M in the cell (spectrum a in FIG. 4). CO gas was then introduced and the spectral change was observed (spectrum b in FIG. 4). NaCN (0.2 mM) was also added and the spectral change was observed (spectrum c in FIG. 4).

The same experiment was also conducted with switching of the order of CO and NaCN addition. Specifically, the hemoCD-Twins solution synthesized in Example 1 was diluted and the ultraviolet-visible absorption spectrum was measured at a concentration of $4.5 \times 10^{-6}$ M in the cell (spectrum a in FIG. 5). NaCN (0.2 mM) was then added and the spectral change was observed (spectrum b in FIG. 5). CO gas was further introduced and the spectral change was observed (spectrum c in FIG. 5).

When CO is added to hemoCD-Twins, hemoCDP(II) in the iron(II) state bonds with CO. However, hemoCDI(II) continues to undergo autoxidation with dilution, being oxidized to iron(III) and exhibiting strong CN scavenging power. It was demonstrated that the hemoCD-Twins aqueous solution exhibits scavenging ability for both CO and CN, scavenging both regardless of which was added first.

[Test Example 3: Spectral analysis of urine after administration of inclusion complex to mice]

Mice were administered the hemoCDP(II) synthesized in Reference Example 1 (7 mM), the hemoCDI(II) synthesized in Reference Example 2 (7 mM) and the hemoCD-Twins synthesized in Example 1 (14 mM), through the abdominal cavity (200 μL), and the absorption spectrum of urine excreted after 30 minutes was measured. In order to determine the iron(II)/iron(III) ratio in the inclusion complex excreted into the urine, CO gas was introduced and the spectrum was measured. A reducing agent ($Na_2S_2O_4$) was further added and the spectrum was measured.

Based on these three spectra, it is possible to calculate the percentages of oxygen (oxy) complex, CO complex and iron(III) complex (met complex) among the complexes in the solution.

Specifically, since CO gas does not react with iron(III), each complex is detected in the following manner in each spectral measurement.

(a) Initial spectral measurement: Detection of oxy complex, CO complex and met complex
(b) Spectral measurement after CO gas introduction in (a): Detection of CO complex and met complex
(c) Spectral measurement after addition of reducing agent in (b): Detection of CO complex

The percentage of met complex can be calculated by the spectral change from (b) to (c), and the percentages of oxy complex and CO complex can be calculated by the spectral change from (a) to (b).

The measurement results for the three spectra of the hemoCDP(II) synthesized in Reference Example 1, the hemoCDI(II) synthesized in Reference Example 2 and the hemoCD-Twins synthesized in Example 1 are shown in FIGS. 6 to 8, along with the calculated values for the percentage of each complex.

The percentage of complex excreted as iron(II) was 83% with the hemoCDP(II) of Reference Example 1 and 12% with the hemoCDI(II) of Reference Example 2, which were results corresponding to each autoxidation rate, demonstrating that the state able to bond to CO and cyanide ions was excreted as the main component. The hemoCD-Twins mixture was 49% iron(II) and 51% iron(III), indicating that iron(II) and iron(III) were excreted as a mixture, in an effective state for simultaneous detoxification of CO/HCN.

[Test Example 4: Verification of urinary excretion of detoxifying agent in rats]

Rats with body weight of 300 ±15 g were administered the hemoCD-Twins of Example 1 (3.5 mM, 2 mL) through the femoral vein over a period of 30 minutes, and the urinary excretion was subsequently quantified by the ultraviolet-visible absorption spectrum. FIG. 9 shows the urinary excretion amounts each hour, and FIG. 10 shows the

cumulative values for urinary excretion.

The results shown in FIGS. 9 and 10 demonstrate that about 80% of hemoCD-Twins is excreted into the urine by 3 hours after administration.

[Test Example 5: Measurement of changes in heartbeat and blood of rats after administration of detoxifying agent 1]

Rats with body weight of 300 ±15 g were administered the hemoCD-Twins of Example 1 (3.5 mM, 2 mL) through the femoral vein over a period of 30 minutes, and the heart rate and blood pressure thereafter were measured using "Bio Amps" (ADInstruments). The results are shown in FIGS. 11 and 12.

Administration was at the 0 min time point, and since a slight change in both was seen immediately after administration but quickly followed by return to steady states for heartbeat and blood pressure, this demonstrated that administration of hemoCD-Twins does not affect heartbeat or blood pressure.

[Test Example 6: Measurement (1) of changes in detoxifying agent concentration in blood of rats after administration of detoxifying agent 1]

Rats with body weight of 300 ±15 g were administered the hemoCD-Twins of Example 1 (3.5 mM, 2 mL) through the femoral vein over a period of 30 minutes, and 200 pL of blood was collected through the cervical vein at 10, 20, 30, 40, 50, 60, 75, 90, 120, 150 and 180 minutes. The obtained blood samples were centrifuged at 12,000 rpm for 5 minutes at 4°C, and 100 μL was taken from the plasma in the supernatant. A 50 μL portion of methanol was added to the plasma to precipitate the plasma proteins. After standing for 15 minutes at 4°C, it was then centrifuged at 12,000 rpm for 15 minutes. Next, 100 μL was skimmed from the supernatant and diluted in 1 mL of PBS (phosphate buffer), after which sodium dithionite ($Na_2S_2O_4$) and carbon monoxide were added to the solution to prepare an analysis sample. Change in blood concentration thereafter was measured by the absolute calibration curve method using HPLC (size exclusion column). The measurement was by analysis carried out using an Erich SEC70 10 × 300 gel filtration column at a flow rate of 0.5 mL/min, and elution was confirmed with an absorber at 422 nm. The results are shown in FIGS. 13 and 14.

The results in FIGS. 13 and 14 demonstrated rapid increase and decrease in blood concentration, and rapid clearance from the blood into the urine.

[Test Example 7: Verification of CO and HCN toxicity in mice]

With mice as the experimental animals, a group administered 0.15 mg of NaCN (oral) and 5000 ppm of CO (n = 10), a group administered 0.15 mg of NaCN alone (n = 7) and a group administered 5000 ppm of CO alone (n = 6) were examined for survival rate and behavioral recovery rate following administration, as shown in FIG. 15. The results are shown in FIG. 16 and FIG. 17.

Based on the survival curve shown in FIG. 16, survival rates were high with administration of NaCN alone or with CO alone, but administration of both resulted in death of all of the mice within 30 minutes.

According to the behavioral observation results shown in FIG. 17, behavior recovered from being incapable of action in the case of either CO or NaCN alone, but no recovery from being incapable of action was observed when both were administered.

This confirmed an additive toxicity effect of CO and cyanide.

[Test Example 8: Verification (1) of effect of administering detoxifying agent 1 to CO/HCN poisoned mice]

As shown in FIG. 18, mice were administered 0.15 mg of NaCN (oral) and 5000 ppm of CO, with the hemoCD-Twins synthesized in Example 1 also being intraperitoneally administered (14 mM, 200 ul) after 5 minutes of CO inhalation, and the survival rate and behavioral recovery rate thereafter were examined (n = 9). A group without administration of hemoCD-Twins (n = 10) was used for comparison, with the results shown in FIG. 19 and FIG. 20.

As shown in FIGS. 19 and 20, the mice administered 0.15 mg of NaCN and placed in 5000 ppm CO became incapable of action and died within 30 minutes, whereas approximately 80% of the mice administered hemoCD-Twins 5 minutes after CO inhalation survived (FIG. 19), showing behavioral recovery by 15 minutes after administration (FIG. 20).

Thus, administration of hemoCD-Twins against simultaneous intoxication with CO/HCN can be judged to provide a significant therapeutic effect.

[Test Example 9: Verification (2) of effect of administering detoxifying agent 1 to CO/HCN poisoned mice]

A test was conducted in the same manner as Test Example 8, except that the NaCN dose was 0.1 mg (FIG.

21). In this case, the mortality rate also fell in the non-administered group (FIG. 22), but loss of action ability was prolonged (FIG. 23). All of the mice in the hemoCD-Twins-administered group survived and exhibited rapid behavioral recovery, thus indicating a rapid therapeutic effect against loss of action ability due to CO/HCN poisoning.

[Test Example 10: Observation (1) after administration of detoxifying agent 1 to CO/HCN simultaneous intoxication rat model]

Rats with simultaneous intoxication by administration of 0.15 mg of NaCN (oral) and 5000 ppm of CO in the same manner as Test Example 7 were administered the hemoCD-Twins of Example 1 (14 mM, 2 mL) through the femoral vein over a period of 30 minutes, and the blood pressure thereafter was measured using "Bio Amps" (ADInstruments). Rats with body weight of 300 ±15 g were administered PBS (2 mL) through the femoral vein over a period of 30 minutes, and the blood pressure thereafter was measured using "Bio Amps"

(ADInstruments).

The time-dependent changes in blood pressure of the rats administered hemoCD-Twins and the rats administered PBS are shown in FIG. 24.

As shown in FIG. 24, rats simultaneously intoxicated with CO/HCN had rapidly lowered blood pressure due to simultaneous intoxication, but the rats administered hemoCD-Twins were able to promptly return to steady state blood pressure due to the administration of hemoCD-Twins.

[Test Example 11: Observation after administration of detoxifying agent 1 to CO simple intoxication rat model]

Using a group of rats administered 2000 ppm of CO as experimental animals (n = 3), the time-dependent change in CO-Hb (carbon monoxide-hemoglobin) in the blood after administration of hemoCD-Twins was compared with the time-dependent change in CO-Hb in the blood with administration of PBS instead of hemoCD-Twins for comparison, producing the results shown in FIG. 25.

FIG. 25 shows that hemoCD-Twins also exhibits a detoxifying effect against rats with CO simple intoxication.

[Test Example 12: Observation after administration of detoxifying agent 1 to CN simple intoxication rat model]

Using a group of rats administered 5 mg of NaCN per 1 kg body weight as experimental animals (n = 3), the time-dependent change in CN- concentration in the blood after administration of hemoCD-Twins was compared with the time-dependent change in CN- concentration in the blood with administration of PBS instead of hemoCD-Twins for comparison, producing the results shown in FIG. 26.

FIG. 26 shows that hemoCD-Twins also exhibits a detoxifying effect against rats with CN simple intoxication.

[Test Example 13: Blood concentration of hemoCD-Twins]

2 ML of a PBS solution containing 3.5 mM hemoCD-Twins was administered to rats (mean: 300 g, isoflurane anesthesia) through the femoral vein over a period of 30 minutes. Blood was then sampled at fixed time periods through the carotid artery, and the blood was centrifuged to obtain plasma samples. The hemoCD-Twins in the plasma was quantified using size exclusion chromatography. The quantitation results were plotted against time to obtain the blood concentration profile shown in FIG. 27.

Based on FIG. 27, hemoCD-Twins is almost totally excreted by 100 minutes.

[Test Example 14: Urine clearance of hemoCD-Twins]

2 ML of a PBS solution containing 14 mM hemoCD-Twins was administered to rats (mean: 300 g, isoflurane anesthesia) through the femoral vein over a period of 10 minutes. Urine pooling in the bladder was sampled at fixed time periods thereafter. The hemoCD-Twins in the sampled urine was quantified based on the UV-vis spectrum. The percentage of hemoCD-Twins detected with respect to the administered dose was plotted against urine sampling time, to obtain the urine clearance profile shown in FIG. 28.

Based on FIG. 28, 100% of the hemoCD-Twins administered to the rat blood was excreted in the urine after 120 minutes.

In other words, since hemoCD-Twins entering the body was quantitatively excreted in the urine without being degraded in the body, it did not affect the rat bodies, demonstrating its high safety.

[Test Example 15: Monitoring of blood pressure and heart rate]

2 ML of a PBS solution containing 3.5 mM hemoCD-Twins was administered to rats (mean: 300 g, isoflurane anesthesia) through the femoral vein over a period of 30 minutes. The blood pressure and heart rate of the rats during the period were measured using Bio Amps (ADInstruments Ltd.), with the results shown in FIG. 29.
FIG. 29 shows that, despite a slight change in blood pressure and heart rate immediately following administration, the heart rate and blood pressure promptly returned to steady state levels.
It was thus shown that administration of hemoCD-Twins does not affect heart rate or blood pressure.

[Test Example 16: Evaluation of nephrotoxicity of hemoCD-Twins]

Mice (mean: 20 g, no anesthesia) were administered 0.2 mL of a PBS solution containing 14 mM hemoCD-Twins, through the abdominal cavity. A PBS-administered group (0.2 mL) was also prepared for comparison. After 24 hours, blood was collected from the mice and the blood creatinine concentration was quantified using a LabAssay Creatinine (Fujifilm Wako, Japan).
The results are shown in FIG. 30.
Based on FIG. 30, the blood creatinine levels after hemoCD-Twins administration were essentially unchanged from the creatinine levels after PBS administration, indicating that hemoCD-Twins has no nephrotoxicity and is highly safe.

[Test Example 17: Verification (1) of CO detoxication effect of fifth inclusion complex, as a novel inclusion complex]

In the same manner as Reference Example 1, Fe(III)TPPS and Py3CD were mixed in a molar ratio of 1:1 and added to physiological saline to obtain an aqueous solution containing the novel fifth inclusion complex (central metal $M_2 = Fe^{3+}$).
Next, as shown in FIG. 31, 200 μL of the aqueous solution (novel fifth inclusion complex: 7 mM) was administered to mice through the abdominal cavity 5 minutes after causing them to inhale CO gas (5000 ppm), and urine sampled 30 minutes later was measured in the same manner as Test Example 3. The percentages of CO complex, Fe(II) oxygen complex (oxy) and oxidized Fe(III) (met) were calculated using molar extinction coefficients, with the results shown in FIG. 32.
Based on FIG. 32, approximately 82% of (CO + oxy) had been excreted into the urine as the reduced iron(II) form.
In other words, the fifth inclusion complex is reduced in the body and exhibits a detoxication effect on CO, even when the central metal is trivalent.

[Test Example 18: Verification (2) of CO detoxication effect of fifth inclusion complex, as a novel inclusion complex]

As shown in FIG. 33, after causing inhalation of CO gas (2000 ppm) for 15 minutes, rats were venously administered 2 mL of the aqueous solution after 15 minutes (novel fifth inclusion complex: 10 mM) at a rate of 15 mL/min and the blood CO-Hb level was measured every 10 minutes after administration, and then after causing inhalation of CO gas (2000 ppm), the rats were venously administered 2 mL of PBS after 15 minutes at a rate of 15 mL/min and the blood CO-Hb level was measured every 10 minutes after administration, producing the comparison results shown in FIG. 34.
Based on FIG. 34, the novel fifth inclusion complex exhibits a detoxication effect on CO, even when the central metal is trivalent.
The present inventors first thought that the first inclusion complex hemoCDP(II) has the ability to detoxify CO but that the novel fifth inclusion complex hemoCDP(III) does not have the ability to detoxify CO.
However, Test Examples 15 to 18 above demonstrated that the novel fifth inclusion complex hemoCDP(III) is reduced in the body, allowing it to detoxify CO.
Likewise, it was thought that the sixth inclusion complex hemoCDI(III) has the ability to detoxify cyanide but that the novel second inclusion complex hemoCDI(II) does not have the ability to detoxify cyanide, but it was demonstrated that the novel second inclusion complex hemoCDI(II) is oxidized in the body, allowing it to detoxify cyanide.
Thus, the hemoCD-Twins mixture allows convenient administration of both with conversion to the same valency, allowing it to function as a detoxifying agent that can simultaneously detoxify CO and cyanide in the body.

## Claims

1. A detoxifying agent for removal of carbon monoxide and hydrogen cyanide in the body, which includes as active ingredients a first inclusion complex wherein a first cyclodextrin dimer represented by general formula (1) below

clathrates a first water-soluble metal porphyrin in which the central metal is divalent, and a second inclusion complex wherein a second cyclodextrin dimer represented by general formula (2) below clathrates the first water-soluble metal porphyrin in which the central metal is divalent.

[Chemical Formula 1]

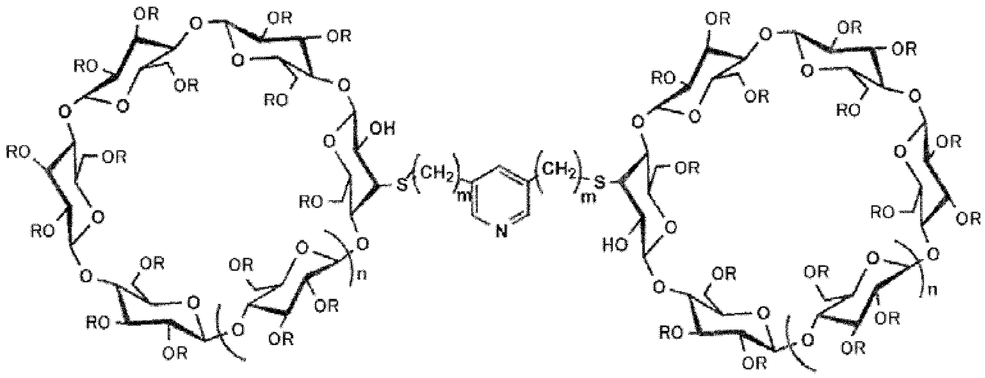

(1)

(In general formula (1) above, each R represents a protecting group protecting a hydroxyl group of cyclodextrin, m represents an integer of 1 to 2, and n represents an integer of 1 to 3.)

[Chemical Formula 2]

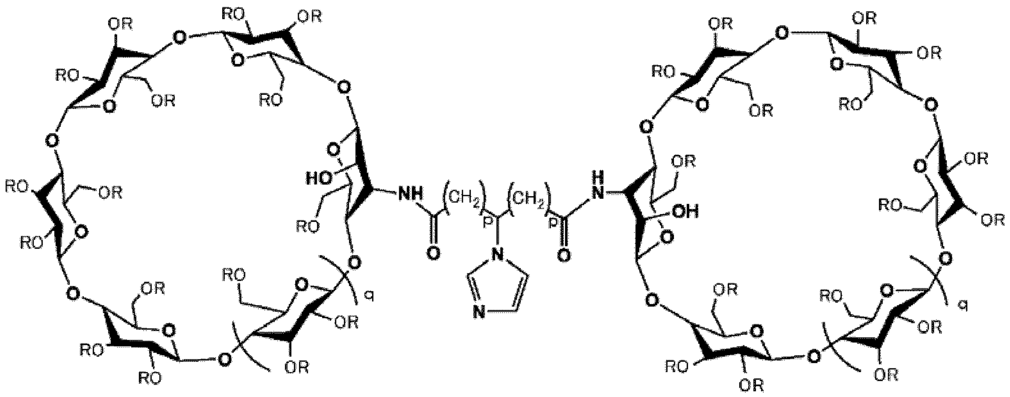

(2)

(In general formula (2) above, each R represents a protecting group protecting a hydroxyl group of cyclodextrin, p represents an integer of 1 to 2, and q represents an integer of 1 to 3.)

2. The detoxifying agent according to claim 1, wherein the first water-soluble metal porphyrin is represented by general formula (3) or (4) below.

[Chemical Formula 3]

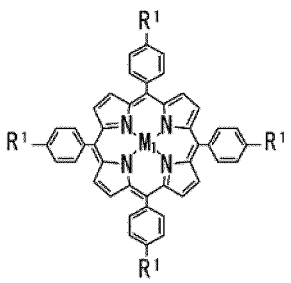

(3)

[Chemical Formula 4]

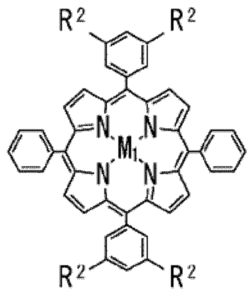

(4)

(In general formulas (3) and (4) above, R1 and R2 each represent a carboxyl, sulfonyl or hydroxyl group, and $M_1$ represents $Fe^{2+}$, $Mn^{2+}$, $Co^{2+}$ or $Zn^{2+}$.)

3. The detoxifying agent according to claim 2, wherein the first water-soluble metal porphyrin is a 5,10,15,20-tetrakis(4-sulfonatophenyl)porphyrin-iron complex.

4. A detoxifying agent kit for preparing the detoxifying agent according to claim 1 or claim 2 for use, which includes an inclusion complex mixture aqueous solution containing a third inclusion complex wherein a first cyclodextrin dimer represented by general formula (1) above clathrates a water-soluble metal porphyrin in which the central metal is divalent or greater, and a fourth inclusion complex wherein a second cyclodextrin dimer represented by general formula (2) above clathrates a water-soluble metal porphyrin in which the central metal is divalent or greater, or a starting material for the same, and a reducing agent.

5. A novel inclusion complex wherein a cyclodextrin dimer represented by general formula (5) below clathrates a first water-soluble metal porphyrin in which the central metal is divalent.

[Chemical Formula 5]

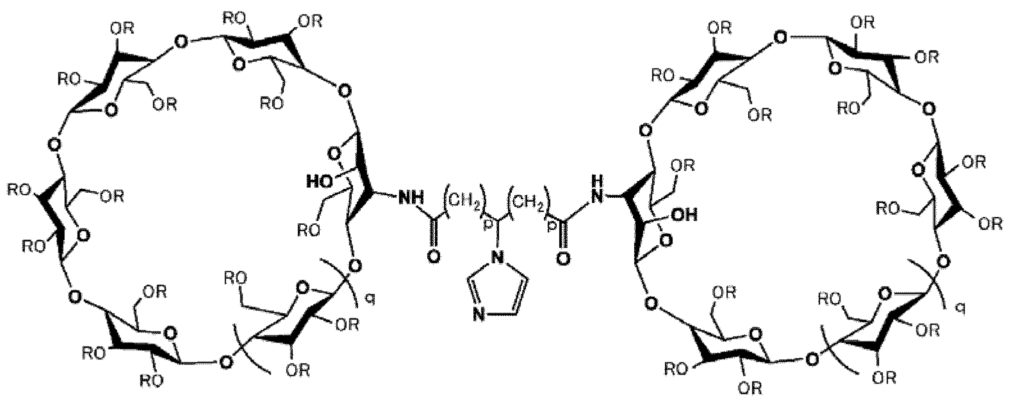

(5)

6. The novel inclusion complex according to claim 5, wherein the first water-soluble metal porphyrin is represented by general formula (6) or (7) below.

[Chemical Formula 6]

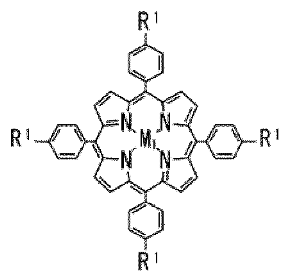

(6)

[Chemical Formula 7]

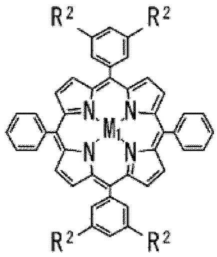

(7)

(In general formulas (6) and (7) above, R1 and R2 each represent a carboxyl, sulfonyl or hydroxyl group, and $M_1$ represents $Fe^{2+}$, $Mn^{2+}$, $Co^{2+}$ or $Zn^{2+}$.)

**7.** The novel inclusion complex according to claim 6, wherein the first water-soluble metal porphyrin is a 5,10,15,20-tetrakis(4-sulfonatophenyl)porphyrin-iron complex.

**8.** A detoxifying agent for removal of carbon monoxide and hydrogen cyanide in the body, which includes as active ingredients a fifth inclusion complex wherein a first cyclodextrin dimer represented by general formula (8) below clathrates a second water-soluble metal porphyrin in which the central metal is trivalent, and a sixth inclusion complex wherein a second cyclodextrin dimer represented by general formula (9) below clathrates the second water-soluble metal porphyrin in which the central metal is trivalent.

[Chemical Formula 8]

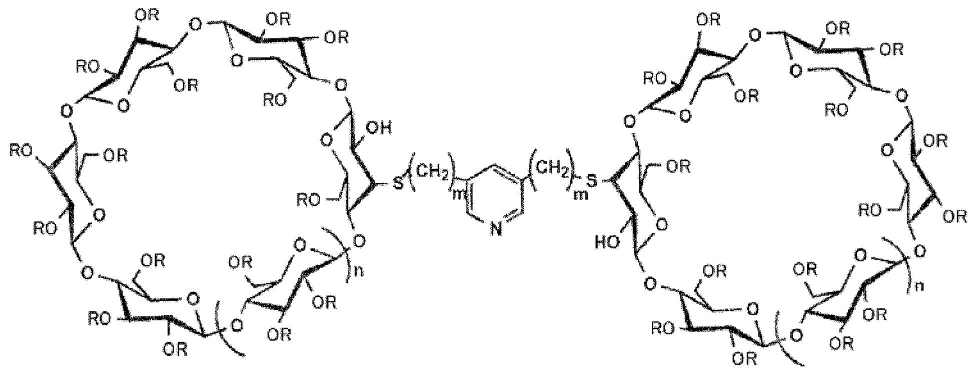

(8)

(In general formula (8) above, each R represents a protecting group protecting a hydroxyl group of cyclodextrin, m represents an integer of 1 to 2, and n represents an integer of 1 to 3.)

[Chemical Formula 9]

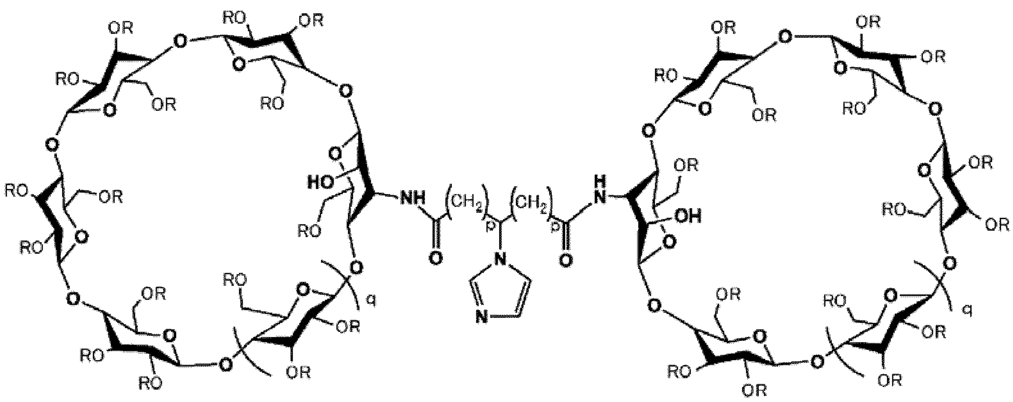

(9)

(In general formula (9) above, each R represents a protecting group protecting a hydroxyl group of cyclodextrin, p

represents an integer of 1 to 2, and q represents an integer of 1 to 3.)

**9.** The detoxifying agent according to claim 8, wherein the second water-soluble metal porphyrin is represented by general formula (10) or (11) below.

[Chemical Formula 10]

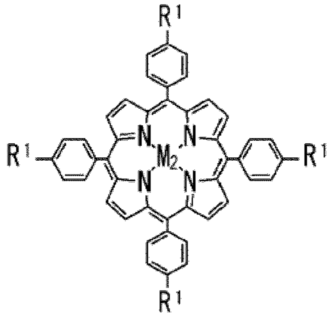

(10)

[Chemical Formula 11]

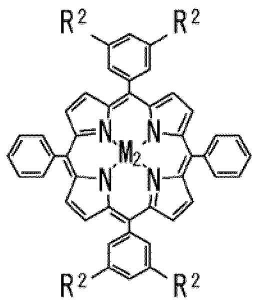

(11)

In general formulas (10) and (11) above, R1 and R2 each represent a carboxyl, sulfonyl or hydroxyl group, and $M_2$ represents $Fe^{3+}$ or $Co^{3+}$.

**10.** The detoxifying agent according to claim 9, wherein the second water-soluble metal porphyrin is a 5,10,15,20-tetrakis(4-sulfonatophenyl)porphyrin-iron complex.

**11.** A detoxifying agent kit for preparing the detoxifying agent according to claim 8 or claim 9 for use,
which includes an inclusion complex mixture aqueous solution containing a fifth inclusion complex wherein the first cyclodextrin dimer represented by general formula (8) above clathrates the second water-soluble metal porphyrin, and a sixth inclusion complex wherein the second cyclodextrin dimer represented by general formula (9) above clathrates the second water-soluble metal porphyrin, or a starting material for the same, and a reducing agent.

**12.** A novel inclusion complex wherein a cyclodextrin dimer represented by general formula (12) below clathrates a second water-soluble metal porphyrin in which the central metal is trivalent.

[Chemical Formula 12]

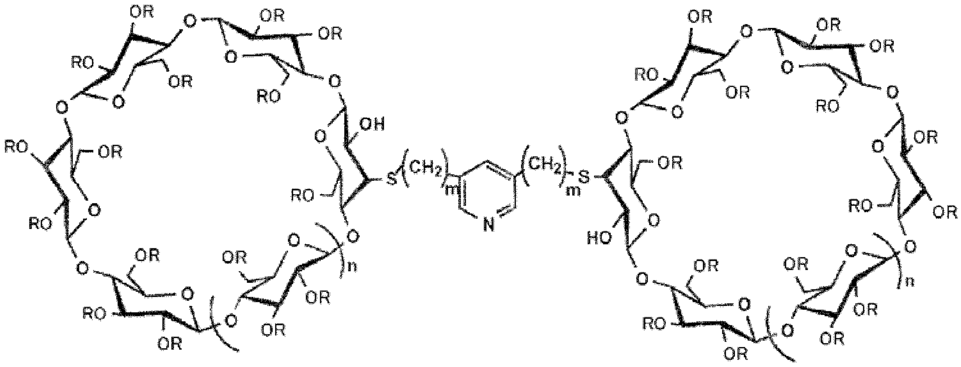

(12)

13. The novel inclusion complex according to claim 12, wherein the second water-soluble metal porphyrin is represented by general formula (13) or (14) below.

[Chemical Formula 13]

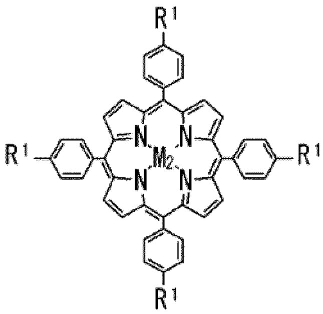

(13)

[Chemical Formula 14]

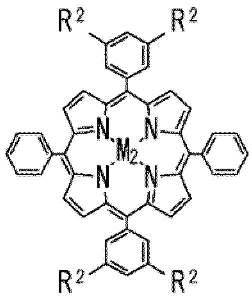

(14)

(In general formulas (13) and (14) above, R1 and R2 each represent a carboxyl, sulfonyl or hydroxyl group, and $M_2$ represents $Fe^{3+}$ or $Co^{3+}$.)

14. The novel inclusion complex according to claim 13, wherein the second water-soluble metal porphyrin is a 5,10,15,20-tetrakis(4-sulfonatophenyl)porphyrin-iron complex.

FIG. 1

hemoCDP(II) (4.0 × $10^{-6}$ M concentration in cell)

Autoxidation reaction: 37°C in PBS

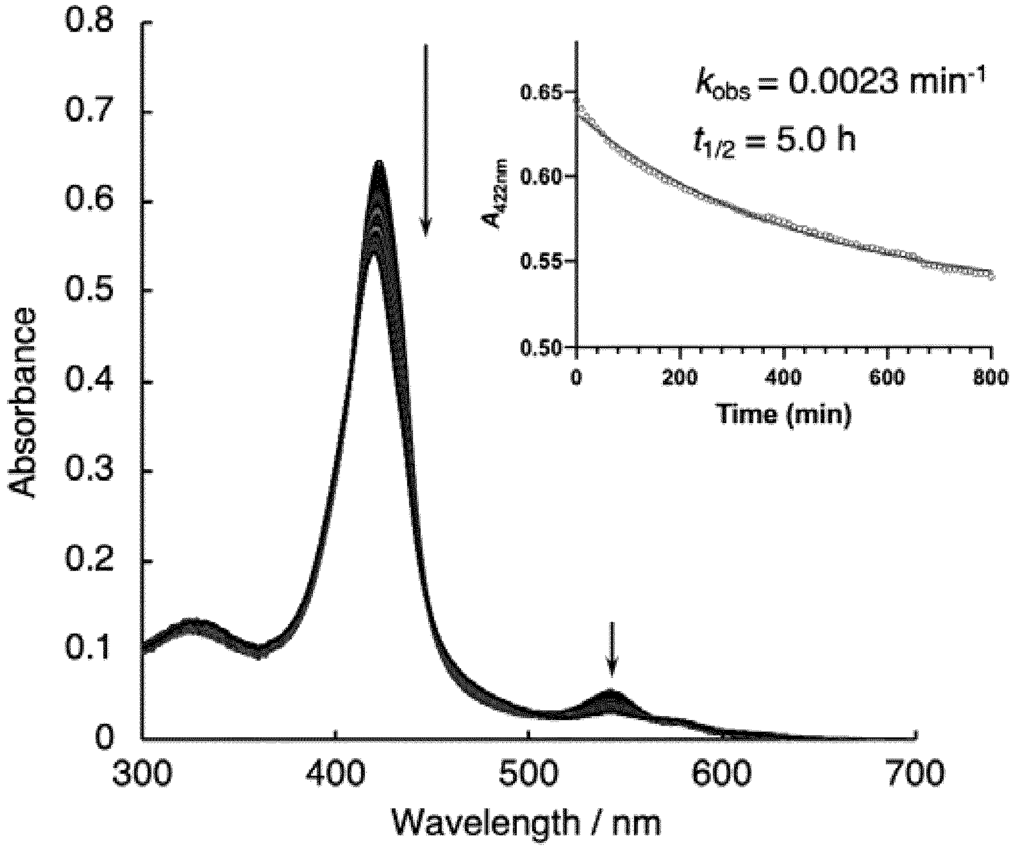

FIG. 2

hemoCDI(II) (3.0 × $10^{-6}$ M concentration in cell)

Autoxidation reaction: 37°C in PBS

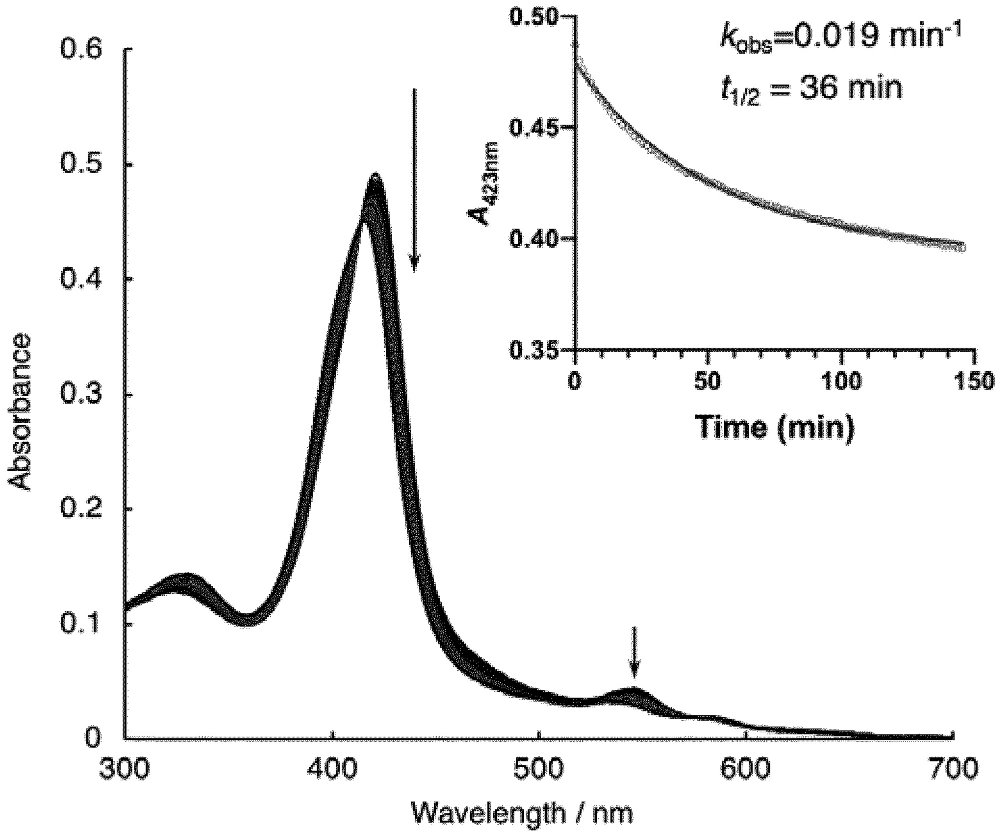

FIG. 3

hemoCD-Twins (6.0 × $10^{-6}$ M concentration in cell)

Autoxidation reaction: 37℃ in PBS

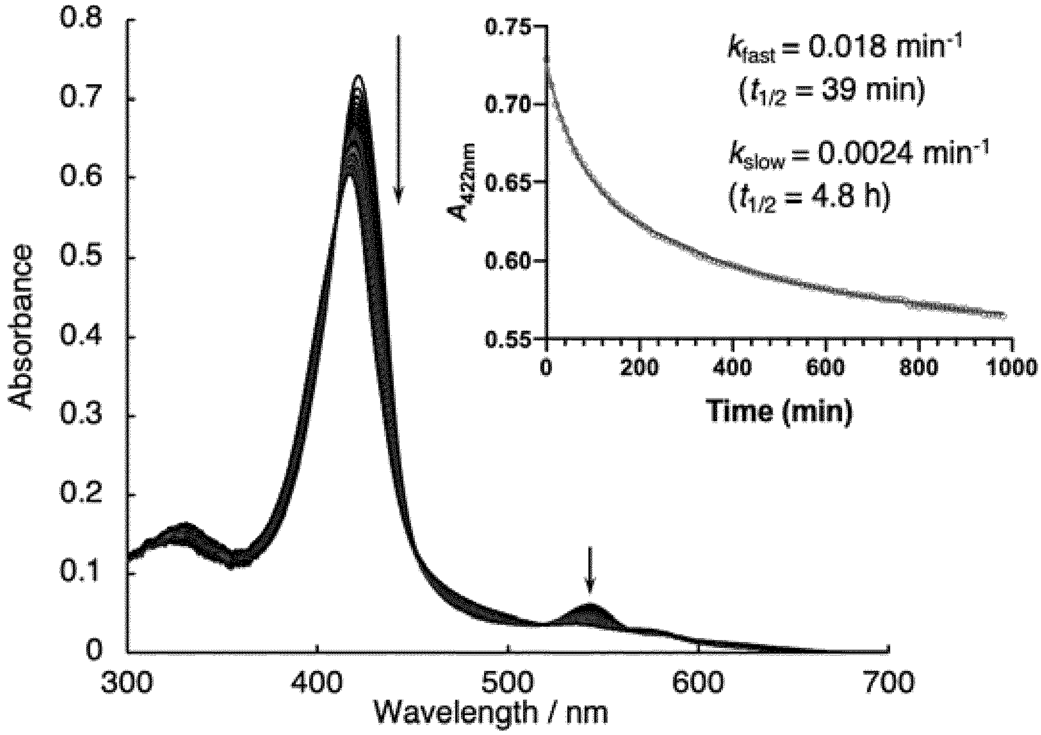

FIG. 4

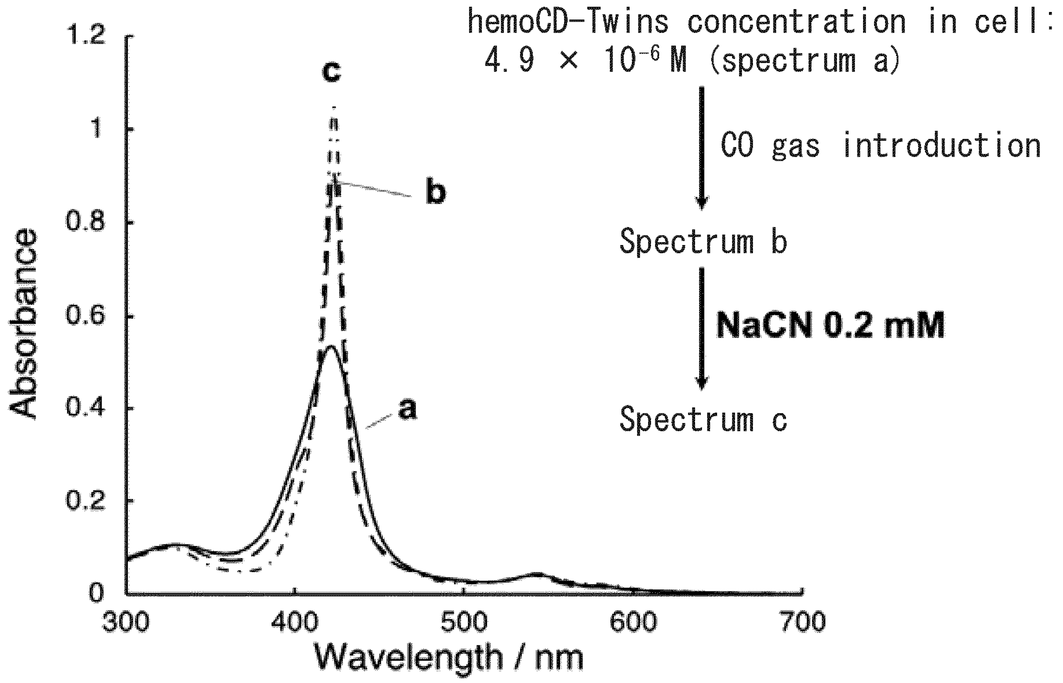
hemoCD-Twins concentration in cell:
4.9 × 10⁻⁶ M (spectrum a)
CO gas introduction
Spectrum b
NaCN 0.2 mM
Spectrum c
Absorbance
Wavelength / nm
a
b
c

FIG. 5

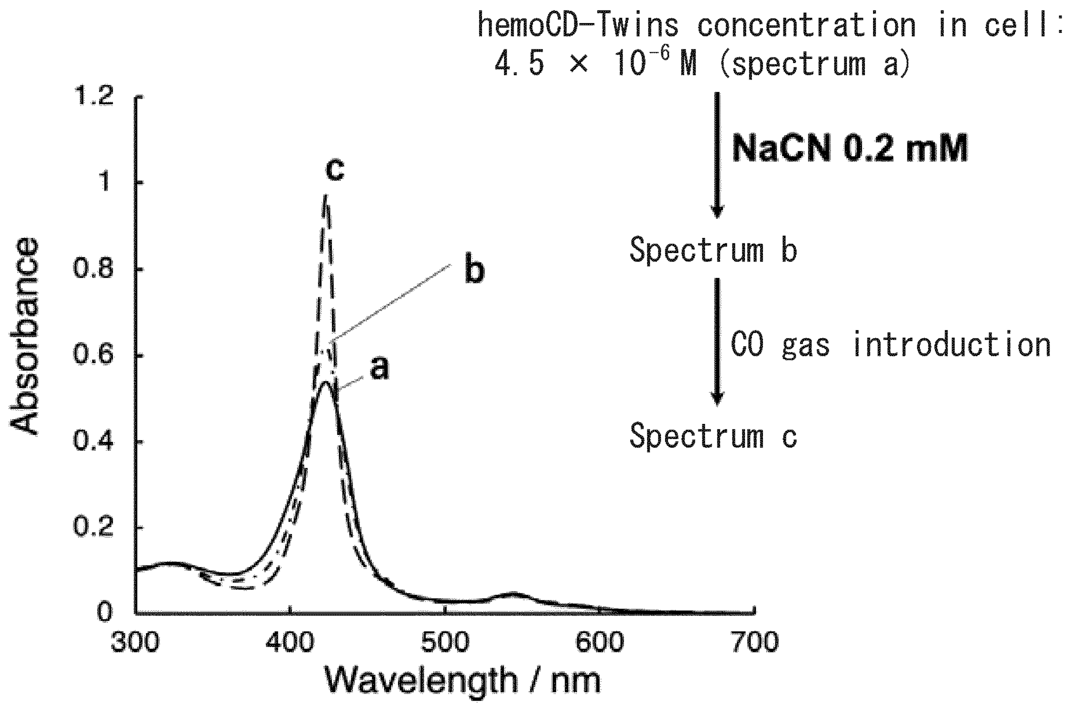
hemoCD-Twins concentration in cell:
4.5 × 10⁻⁶ M (spectrum a)
NaCN 0.2 mM
Spectrum b
CO gas introduction
Spectrum c
Absorbance
Wavelength / nm
a
b
c

FIG. 6

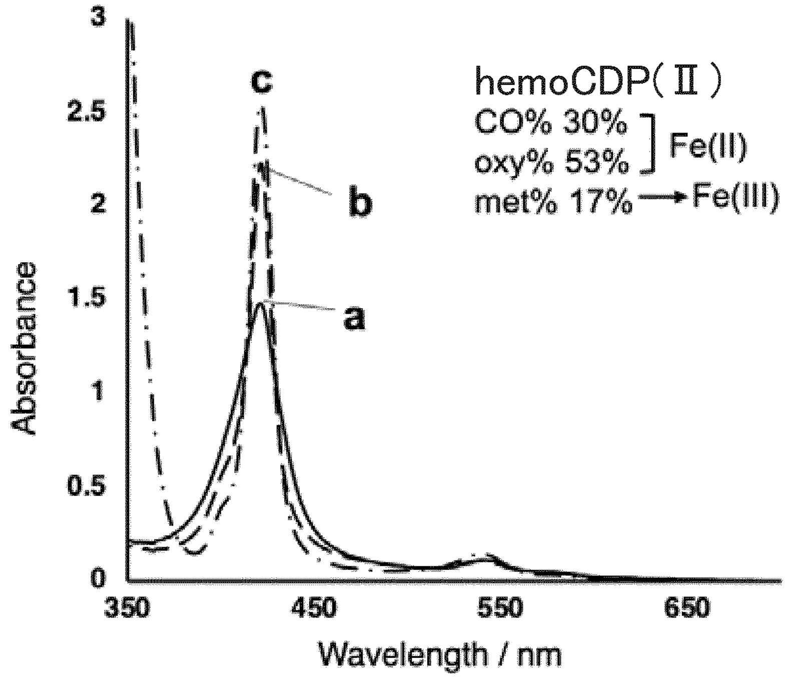
hemoCDP(Ⅱ)
CO% 30%
oxy% 53%
Fe(II)
met% 17% → Fe(III)
a
b
c
Absorbance
Wavelength / nm
0
0.5
1
1.5
2
2.5
3
350
450
550
650

FIG. 7

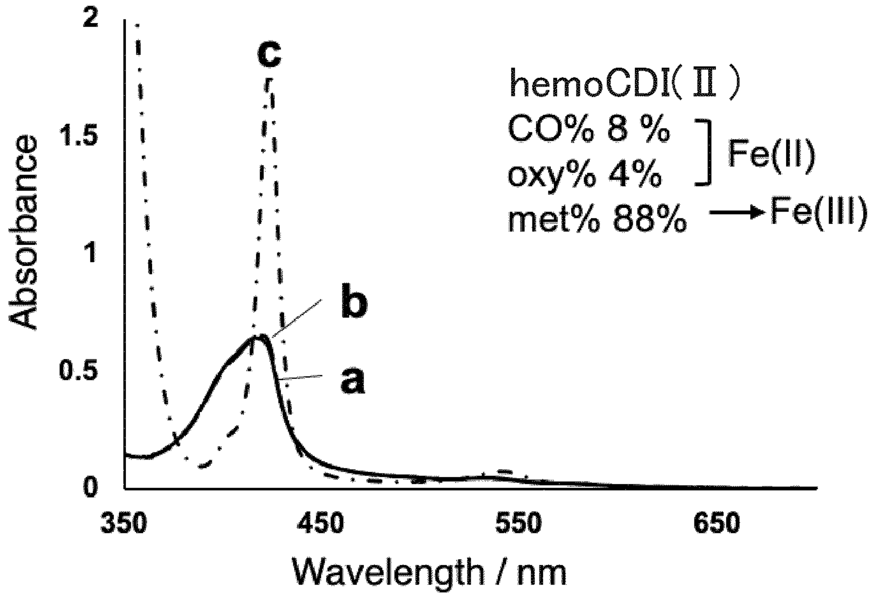
hemoCDI(Ⅱ)
CO% 8 %
oxy% 4%
Fe(II)
met% 88% → Fe(III)
a
b
c
Absorbance
Wavelength / nm
0
0.5
1
1.5
2
350
450
550
650

FIG. 8

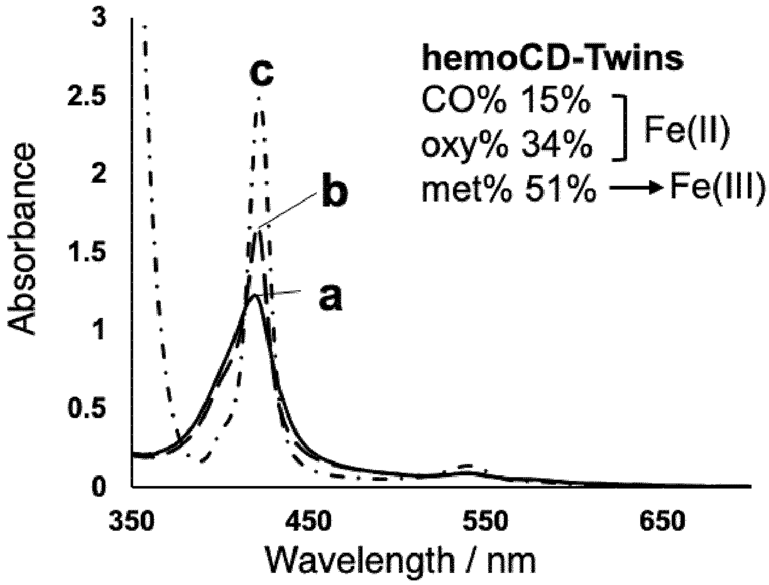
3
2.5
2
1.5
1
0.5
0
Absorbance
c
b
a
hemoCD-Twins
CO% 15%
oxy% 34%
Fe(II)
met% 51%
Fe(III)
350
450
550
650
Wavelength / nm

FIG. 9

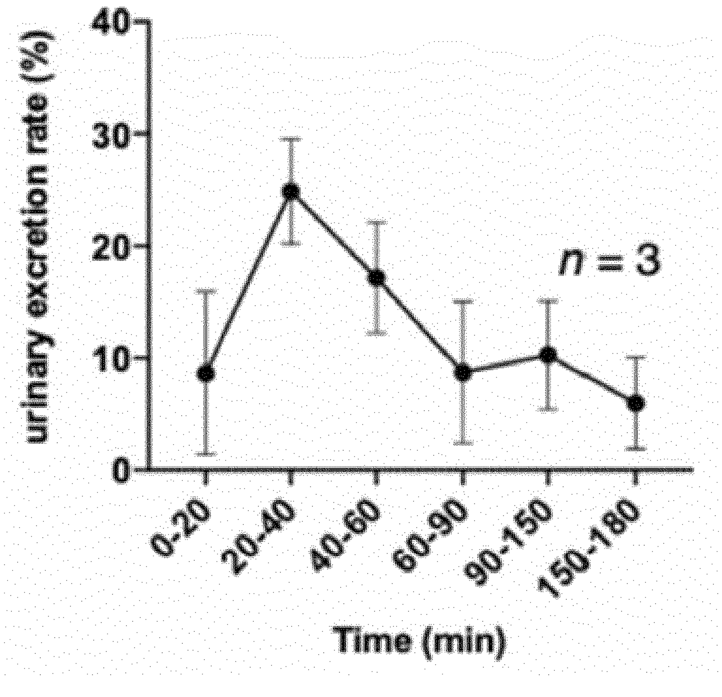
40
30
20
10
0
urinary excretion rate (%)
n = 3
0-20
20-40
40-60
60-90
90-150
150-180
Time (min)

FIG. 10

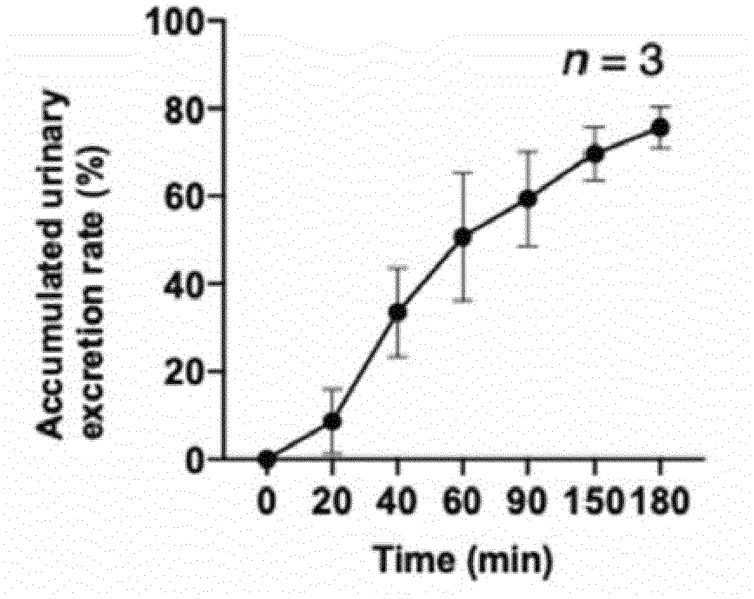


FIG. 11

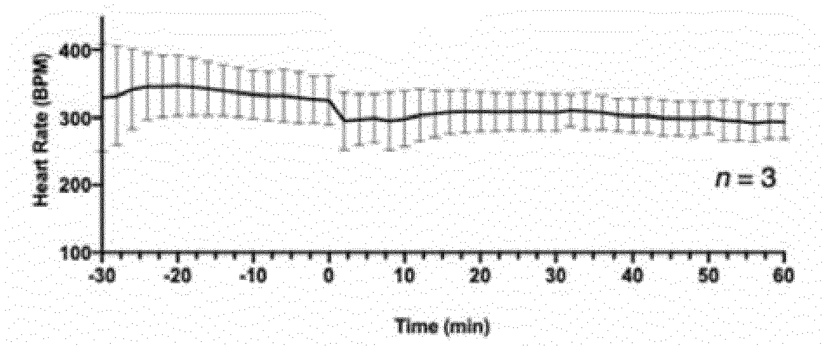


FIG. 12

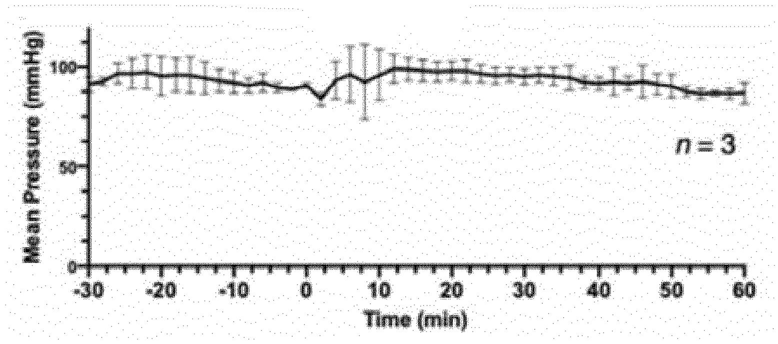

FIG. 13

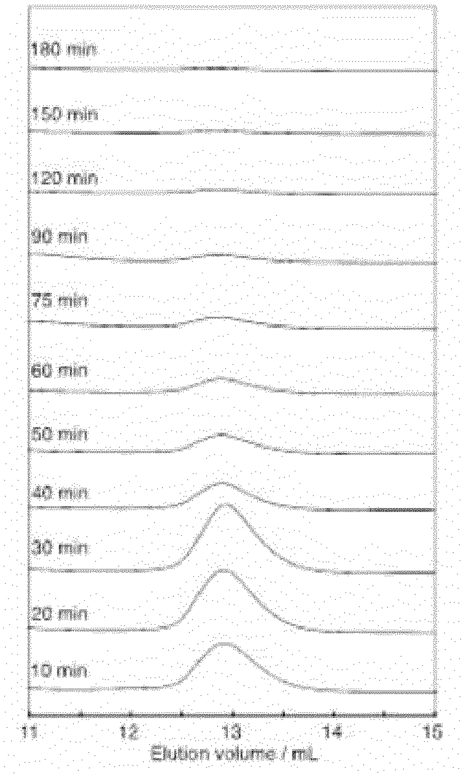
180 min
150 min
120 min
90 min
75 min
60 min
50 min
40 min
30 min
20 min
10 min
11
12
13
14
15
Elution volume / mL

FIG. 14

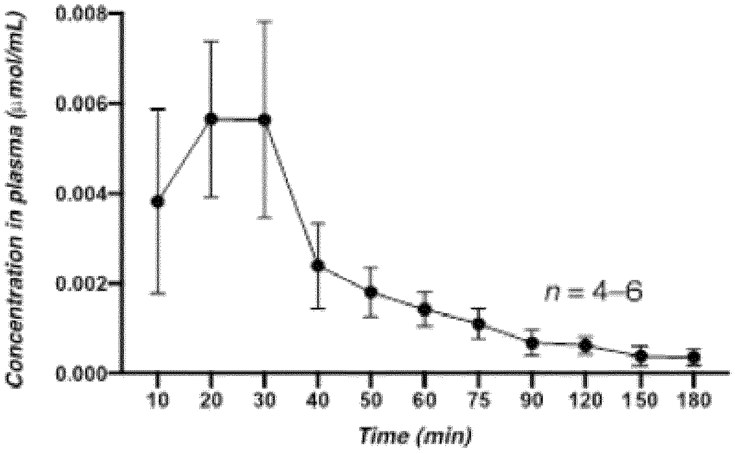
Concentration in plasma (μmol/mL)
0.008
0.006
0.004
0.002
0.000
10
20
30
40
50
60
75
90
120
150
180
Time (min)
n = 4–6

FIG. 15

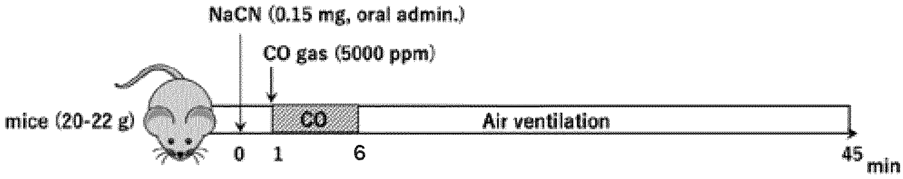


FIG. 16

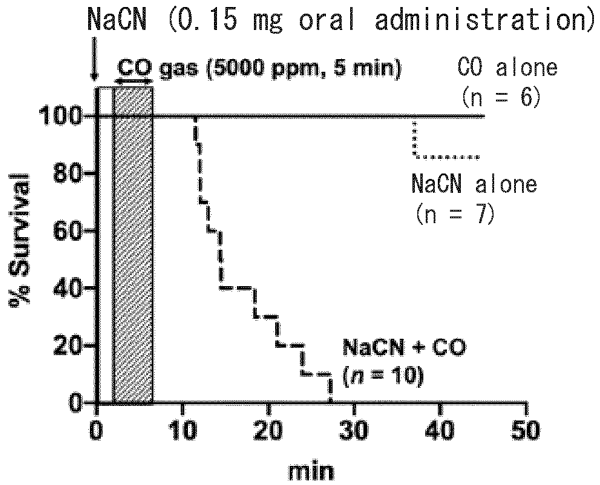


FIG. 17

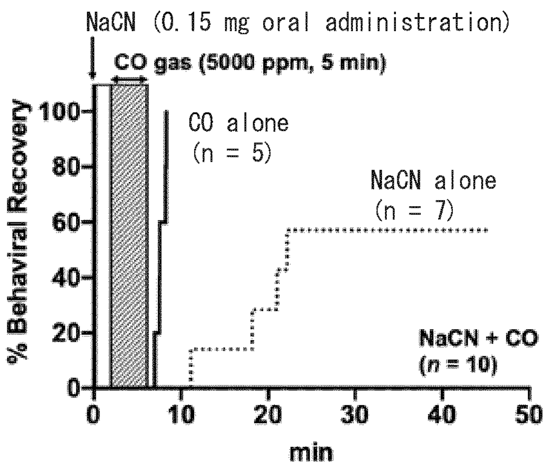

FIG. 18

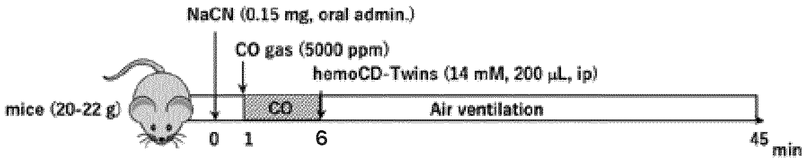


FIG. 19

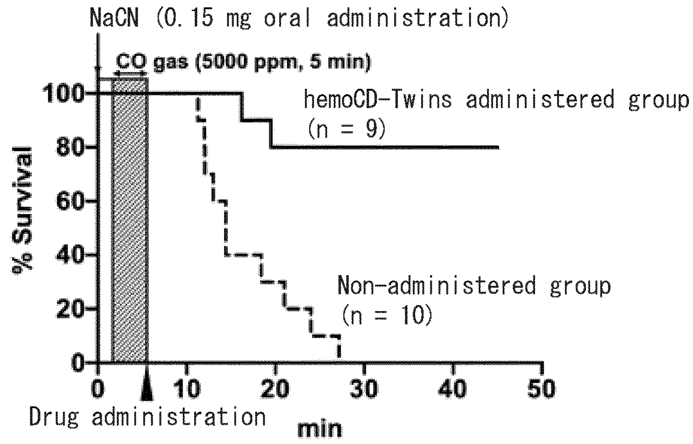


FIG. 20

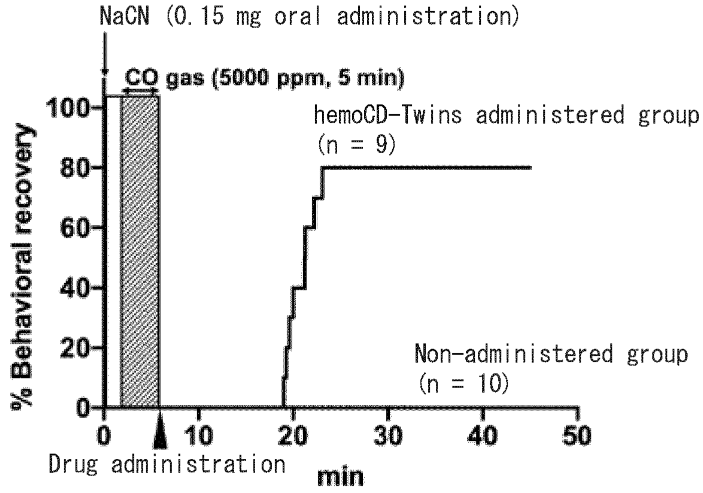

FIG. 21

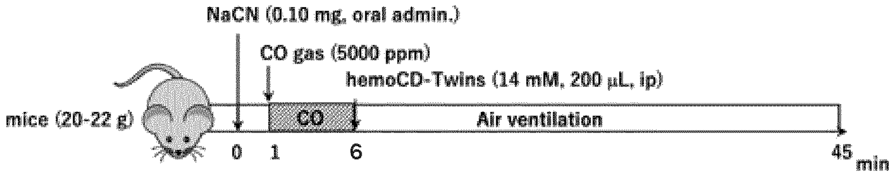


FIG. 22

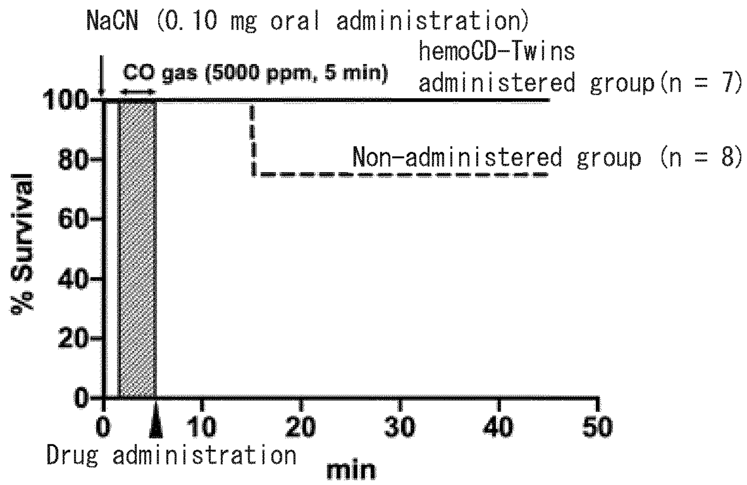


FIG. 23

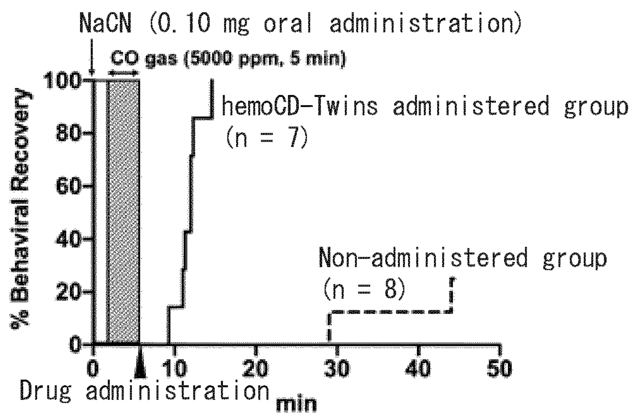

FIG. 24

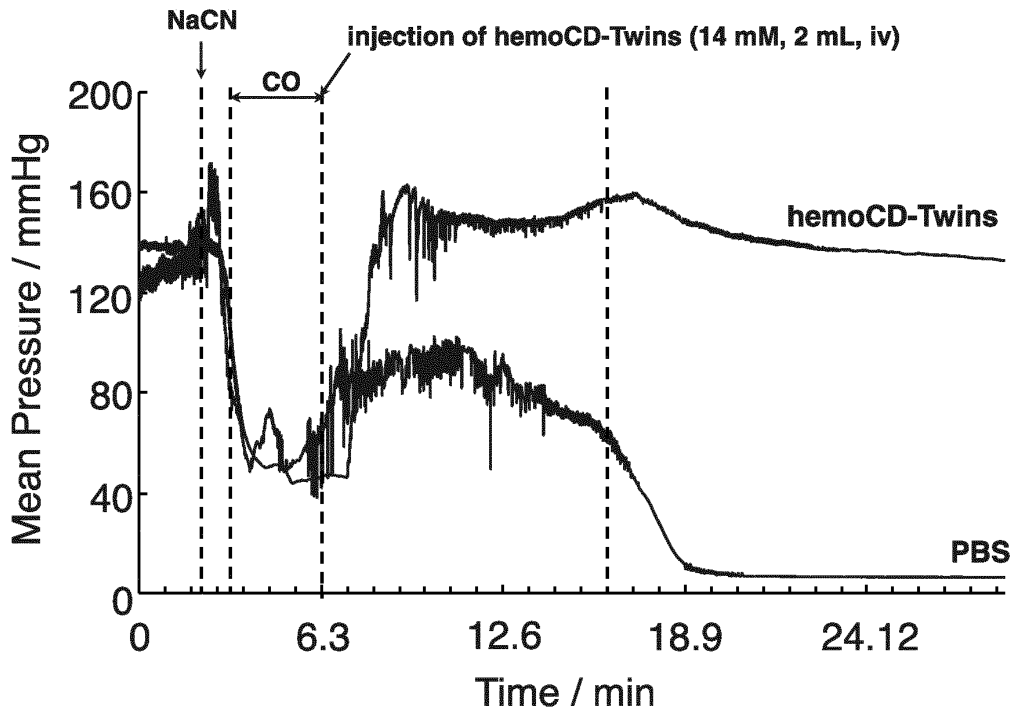
NaCN
CO
injection of hemoCD-Twins (14 mM, 2 mL, iv)
200
160
120
80
40
0
Mean Pressure / mmHg
hemoCD-Twins
PBS
0
6.3
12.6
18.9
24.12
Time / min

FIG. 25

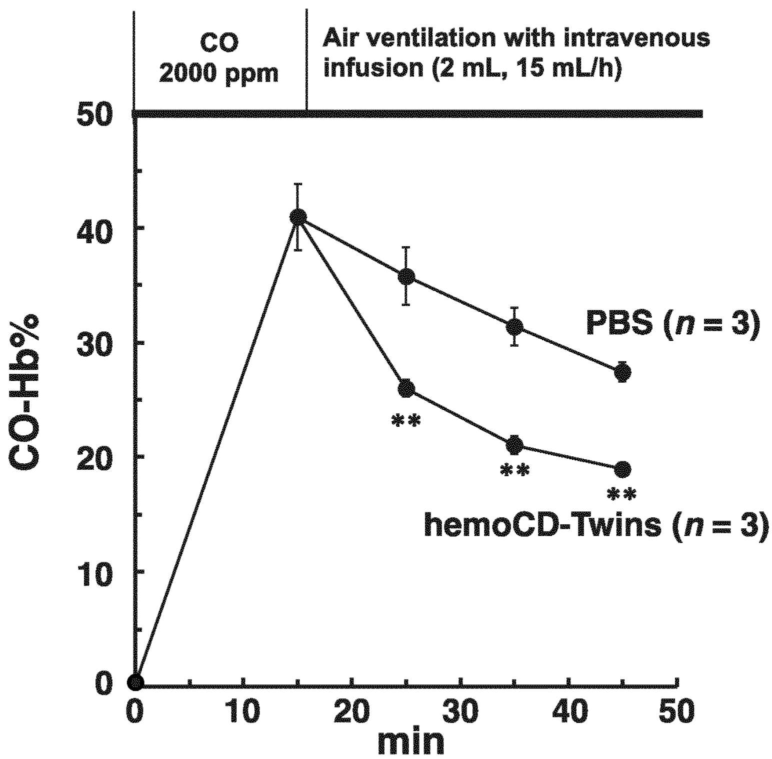
CO
2000 ppm
Air ventilation with intravenous
infusion (2 mL, 15 mL/h)
50
40
30
20
10
0
CO-Hb%
PBS (n = 3)
**
**
**
hemoCD-Twins (n = 3)
0
10
20
30
40
50
min

FIG. 26

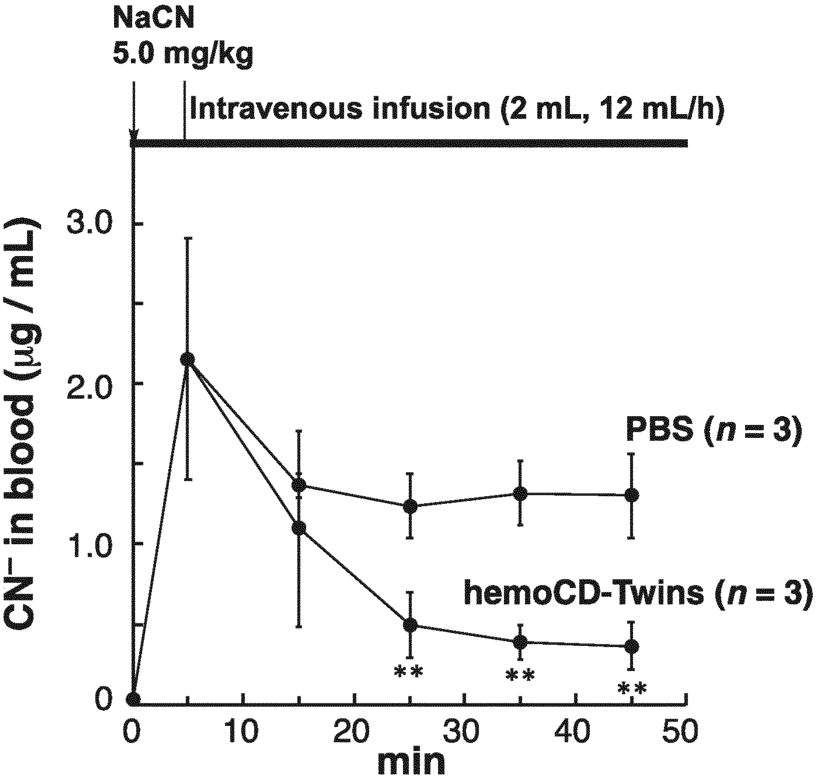
NaCN
5.0 mg/kg
Intravenous infusion (2 mL, 12 mL/h)
CN⁻ in blood (μg / mL)
3.0
2.0
1.0
0
PBS (n = 3)
hemoCD-Twins (n = 3)
**
**
**
0
10
20
30
40
50
min

FIG. 27

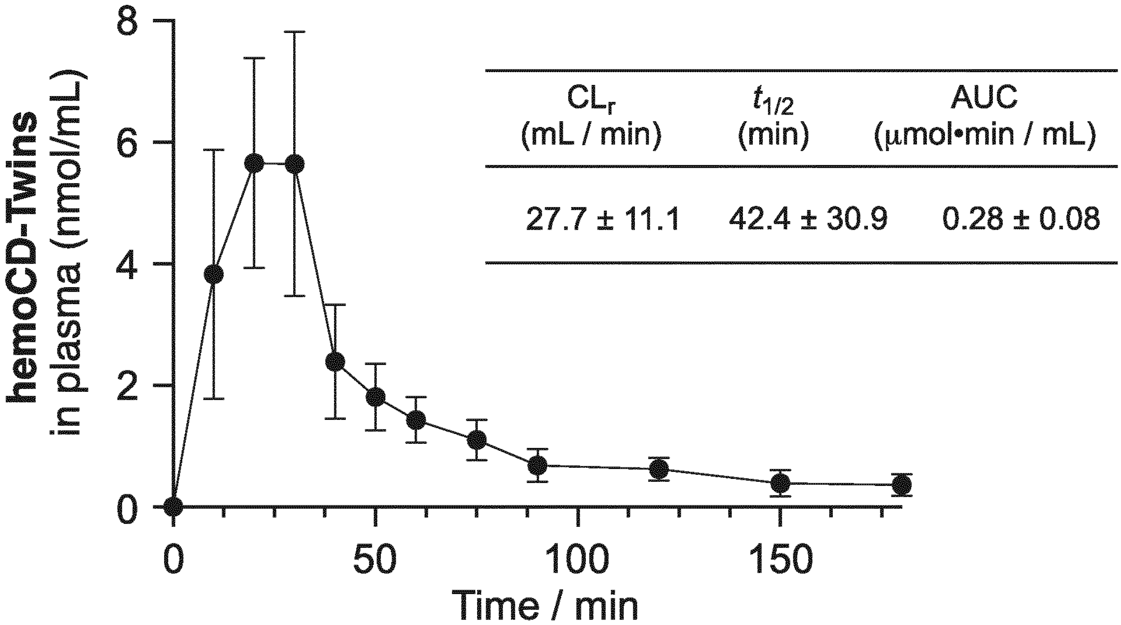
hemoCD-Twins
in plasma (nmol/mL)
8
6
4
2
0
0
50
100
150
Time / min
CLr (mL / min)
t1/2 (min)
AUC (μmol•min / mL)
27.7 ± 11.1
42.4 ± 30.9
0.28 ± 0.08

FIG. 28

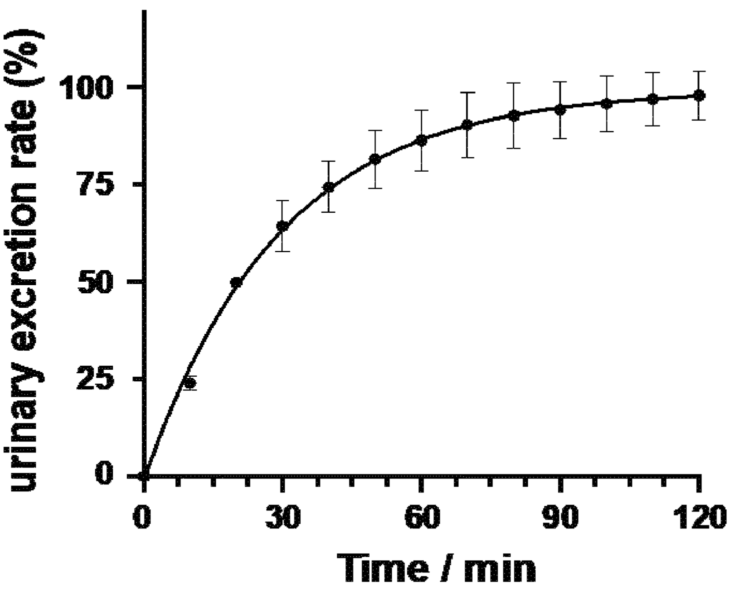
urinary excretion rate (%)
100
75
50
25
0
0
30
60
90
120
Time / min

FIG. 29

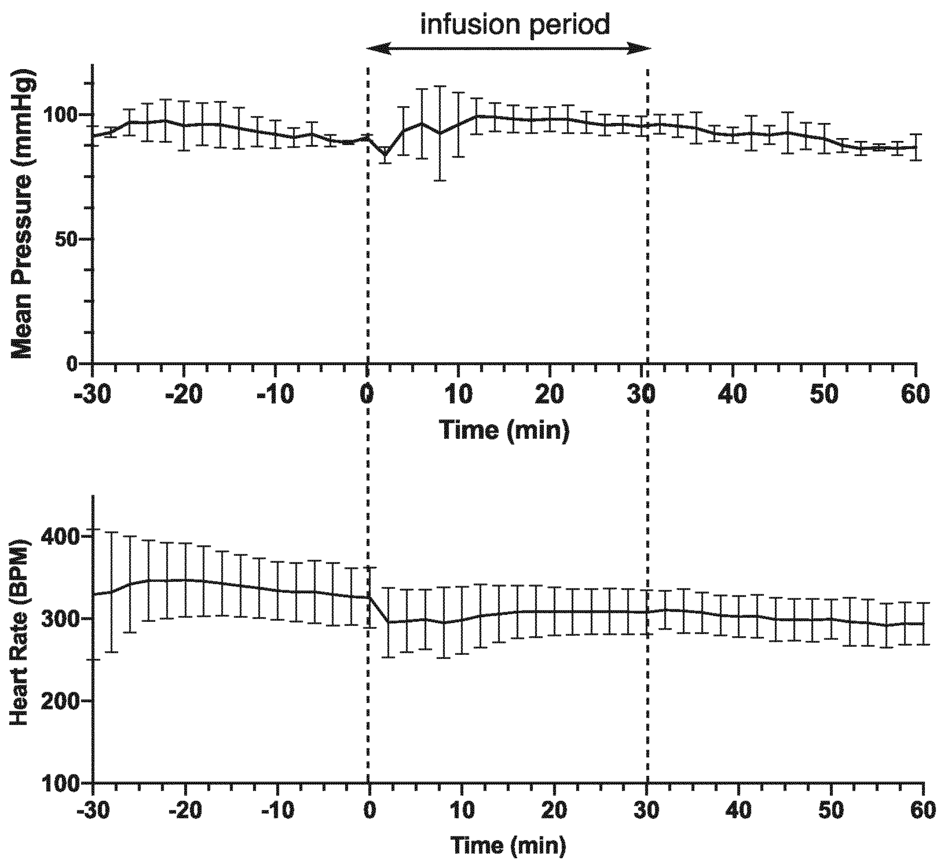
infusion period
Mean Pressure (mmHg)
100
50
0
-30
-20
-10
0
10
20
30
40
50
60
Time (min)
Heart Rate (BPM)
400
300
200
100
-30
-20
-10
0
10
20
30
40
50
60
Time (min)

FIG. 30

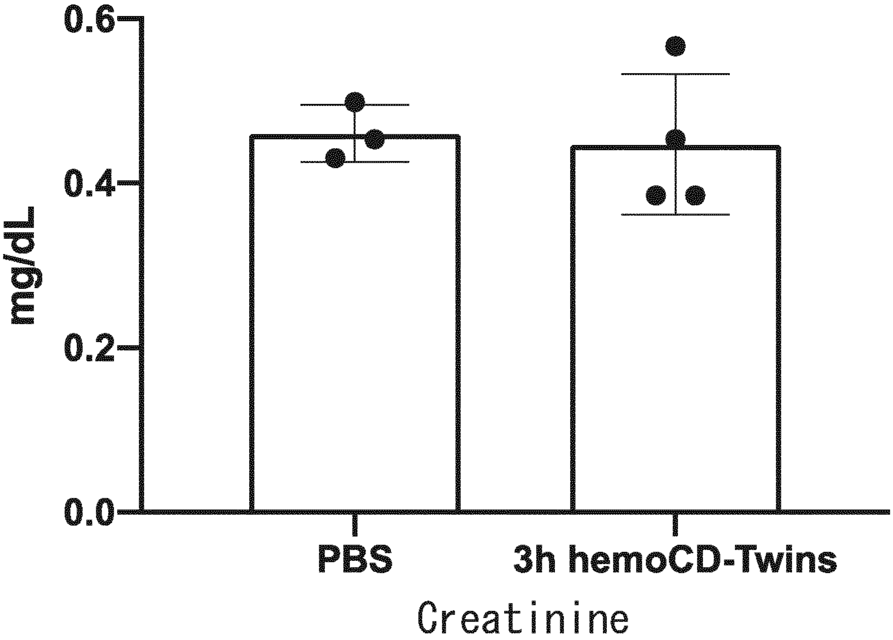

FIG. 31

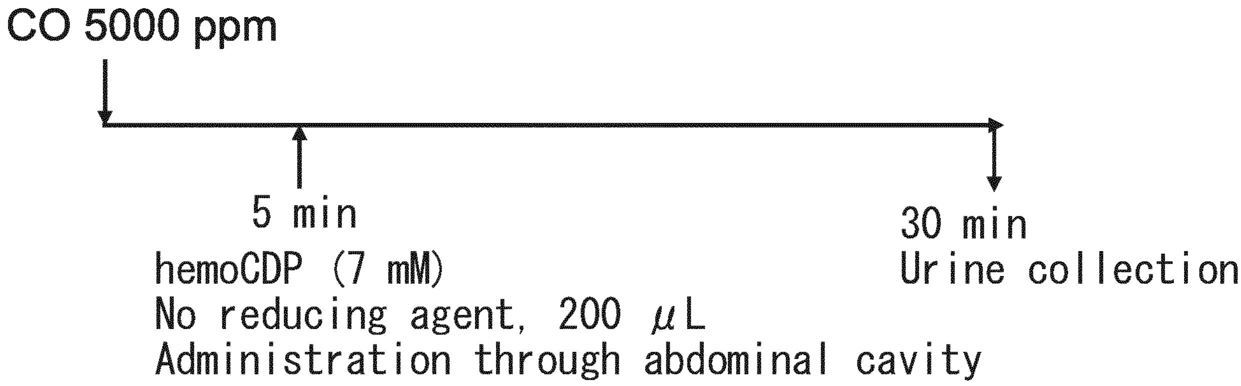
CO 5000 ppm
5 min
hemoCDP (7 mM)
No reducing agent, 200 μL
Administration through abdominal cavity
30 min
Urine collection

FIG. 32

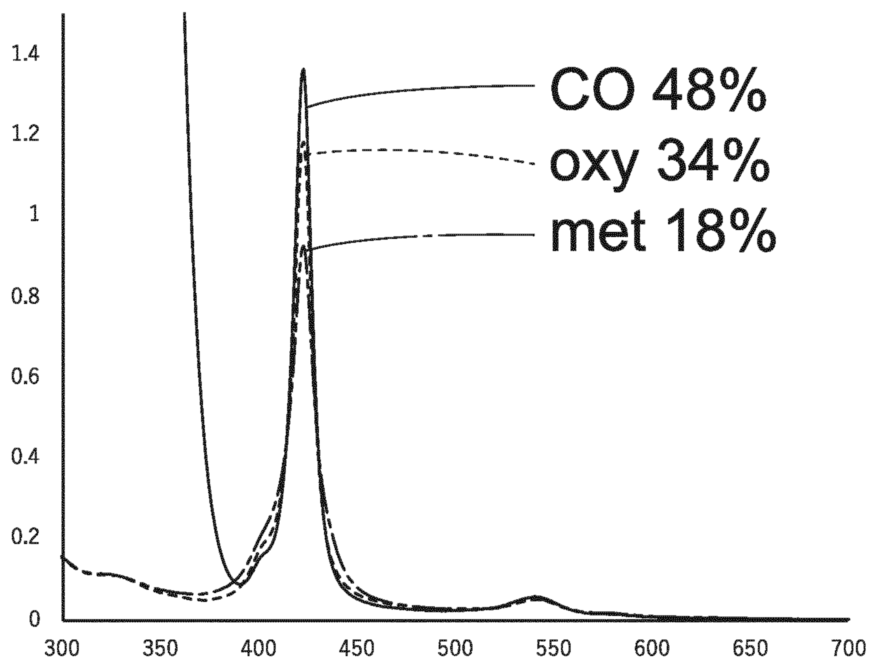
1.4
1.2
1
0.8
0.6
0.4
0.2
0
300
350
400
450
500
550
600
650
700
CO 48%
oxy 34%
met 18%

FIG. 33

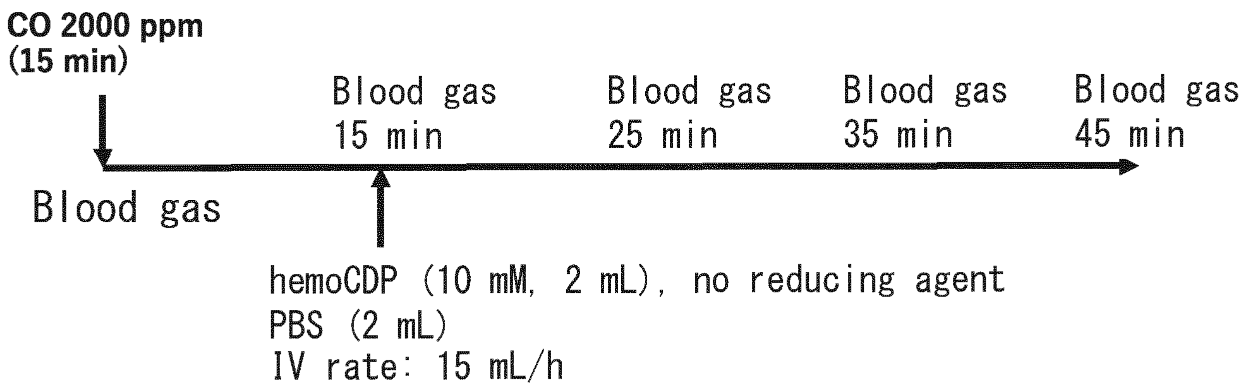
CO 2000 ppm
(15 min)
Blood gas
15 min
Blood gas
25 min
Blood gas
35 min
Blood gas
45 min
Blood gas
hemoCDP (10 mM, 2 mL), no reducing agent
PBS (2 mL)
IV rate: 15 mL/h

FIG. 34

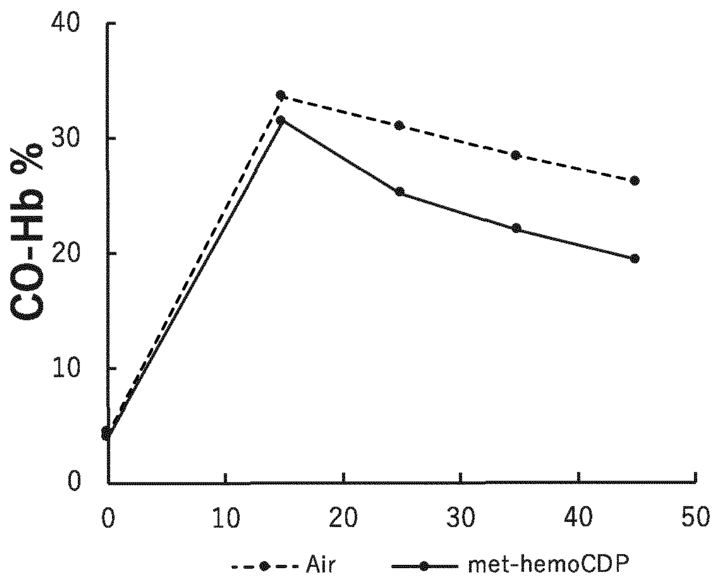
CO-Hb %
40
30
20
10
0
0
10
20
30
40
50
Air
met-hemoCDP

## INTERNATIONAL SEARCH REPORT

International application No.
**PCT/JP2022/038359**

**A. CLASSIFICATION OF SUBJECT MATTER**

***A61K 31/555***(2006.01)i; ***A61K 31/409***(2006.01)i; ***A61K 31/724***(2006.01)i; ***A61K 47/69***(2017.01)i; ***A61P 39/02***(2006.01)i
FI: A61K31/555; A61K31/409; A61K31/724; A61K47/69; A61P39/02

According to International Patent Classification (IPC) or to both national classification and IPC

**B. FIELDS SEARCHED**

Minimum documentation searched (classification system followed by classification symbols)

A61K31/00-31/80; A61K47/69; A61P39/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | KITAGISHI, Hiroaki et al. Synthetic heme protein models that function in aqueous solution. Chemical Communications, 07 January 2021, vol. 57, no. 2, pp. 148-173<br>p. 157, lines 25-27, fig. 12, p. 159, fig. 13, p. 160, scheme 7, left column, line 15, p. 169, right column, lines 7-11, p. 170, left column, lines 5-10, right column, lines 1-4 | 1-7, 12-14<br>8-11 |
| A | JP 2010-194475 A (DOSHISHA) 09 September 2010 (2010-09-09) | 1-14 |
| A | JP 2012-20940 A (DOSHISHA) 02 February 2012 (2012-02-02) | 1-14 |
| A | JP 2013-231111 A (DOSHISHA) 14 November 2013 (2013-11-14) | 1-14 |
| A | US 2016/0000820 A1 (VIRGINIA COMMONWEALTH UNIVERSITY) 07 January 2016 (2016-01-07) | 1-14 |
| A | JP 2019-521966 A (UNIVERSITY OF PITTSBURGH-OF THE COMMONWEALTH SYSTEM OF HIGHER EDUCATION) 08 August 2019 (2019-08-08) | 1-14 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 November 2022** | **06 December 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/038359**

| Patent document cited in search report | Publication date (day/month/year) | Patent family member(s) | Publication date (day/month/year) |
|---|---|---|---|
| JP 2010-194475 A | 09 September 2010 | US 2011/0312914 A1<br>WO 2010/098442 A1<br>EP 2402017 A1 | |
| JP 2012-20940 A | 02 February 2012 | (Family: none) | |
| JP 2013-231111 A | 14 November 2013 | (Family: none) | |
| US 2016/0000820 A1 | 07 January 2016 | (Family: none) | |
| JP 2019-521966 A | 08 August 2019 | US 2019/0290739 A1<br>WO 2017/201447 A1<br>EP 3458038 A1<br>AU 2017267955 A<br>CA 3024171 A<br>CN 109475511 A<br>AU 2020267259 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4803631 B
- JP 2010194475 A
- JP 5619500 B


### Non-patent literature cited in the description


- *Inorg. Chem.,* 2006
- *Chem. Asian J.,* 2006